# EUROPEAN PATENT SPECIFICATION

(11) **EP 2 724 157 B1**
(45) Date of publication and mention of the grant of the patent: **08.03.2017**
(21) Application number: 12735817.4
(22) Date of filing: 27.06.2012
(51) Int. Cl.: G01N 33/50

(54) **METHOD FOR SCREENING CELLS**
SCREENING-VERFAHREN FÜR ZELLEN
PROCÉDÉ DE CRIBLAGE DE CELLULES

(30) Priority: 27.06.2011 EP 11171603
(43) Date of publication of application: 30.04.2014
(73) Proprietor: Valneva, 69007 Lyon (FR)
(72) Inventor: GARRONE, Pierre, F-69008 Lyon (FR); ABÈS, Riad, F-69003 Lyon (FR); BELTRAMINELLI, Nicola, F-69008 Lyon (FR); MEHTALI, Majid, F-44220 Couëron (FR)
(74) Representative: Flesselles, Bruno F.G.
(86) International application number: PCT/EP2012/062523
(87) International publication number: WO 2013/000982

(56) References cited:
- EP-A1- 2 184 345
- WO-A1-2007/068758
- JIN AISHUN ET AL: "A rapid and efficient single-cell manipulation method for screening antigen-specific antibody-secreting cells from human peripheral blood.", NATURE MEDICINE, vol. 15, no. 9, September 2009 (2009-09), pages 1088-1092 + 1, XP002665420, ISSN: 1546-170X
- OZAWA TATSUHIKO ET AL: "Amplification and analysis of cDNA generated from a single cell by 5 '-RACE: application to isolation of antibody heavy and light chain variable gene sequences from single B cells", BIOTECHNIQUES, vol. 40, no. 4, April 2006 (2006-04), page 6PP, XP002665421, ISSN: 0736-6205

## Description

### TECHNICAL FIELD OF THE INVENTION

The invention relates to the field of immunology and more particularly to high throughput screening methods for identifying, isolating and retrieving cells secreting antibodies of interest, e.g., having the desired functional activity and/or multi-antigen specificities. It further relates to a method for the recovery and cloning of said antibodies VH/VL sequences to generate recombinant monoclonal antibodies having the features of interest. The invention further concerns a method for obtaining a cell producing such a monoclonal antibody and a method for producing a monoclonal antibody using said cell.

### BACKGROUND OF THE INVENTION

The design of new potent and safe antibody-based therapies for human diseases is a major objective for pharmaceutical companies. This implies the screening, generation and characterization of a large set of monoclonal antibodies in order to identify antibodies with the desired biological activity and/or with particular binding profiles to the target antigen(s).

Some people naturally develop a strong antibody response against disease-related or self-antigens.

It would thus be suitable to be able to identify, isolate and recover the cells secreting these native human monoclonal antibodies which would have already proven biological activity *in vivo.* The isolation of said antibody secreting cells of interest would enable the subsequent cloning of the genes coding for said antibodies and the production of a recombinant monoclonal antibody, by using well known in vitro recombinant DNA techniques and cell culture methods.

The difficulty to this particular approach is the fact that the antibody secreting cells coding for antibodies with the desired profile (having not only binding properties, but also biological activity), are often memory B cells which are usually present in a very low frequency with regards to the total of blood cells and even to the total of B cells (less than 1/1000 to less than 1/200000).

Accordingly, the identification, isolation and retrieval of cells secreting biologically active antibodies from a biological sample (e.g. blood sample) of a human donor is specially challenging since the frequency of B cells secreting such biologically active antibodies among those that secrete antigen-specific antibodies is usually very low (see for example Pinna et al., Eur J Immunol, 2009, 39: 1260-1270). Consequently, B cells that produce the antibody of interest can be considered as very rare cells present at an extremely low frequency among circulating cells.

Furthermore, these memory B cells do not produce or secrete the antibody of interest without being stimulated (with the specific antigen or other stimulating factors) and their life span is quite low once they have been activated to secrete the antibody.

Several methods for detecting antibody-secreting cells are well known in the art. Typical methods of identifying single antibody-secreting cells comprise the Enzyme-Linked Immunospot (ELISPOT) method (Molecular Biotechnology (2001), Volume: 45, Issue: 2, Pages: 169-171). Recently, new methods for the identification and isolation of B cells comprising a step of high throughput screening of antibody-secreting cells using cell arrays have been described, for example in US 7,776,553, EP1566635, or the ISAAC technology described in EP2184345 and in Jin et al (Nature Protocols, 2011, 6(5) 668-76), aiming to solve the problem of the identification of rare, low frequency, antibody-producing secreting cells.

However, the sensitivity and efficiency of the available methods for the identification of these rare antibody secreting cells producing antibodies with the aimed biological activity and/or multi-antigen specificity and the further cloning of said antibodies are still to be improved. This would allow a reduction of the workload, costs and time necessary for the identification of a new antibody presenting the features of choice.

The yield of recovery of new recombinant antibodies presenting the desired features will depend on the efficiency of the method to (i) identify the B cells presenting the desired functional activity and/or binding profile (directly or by detection of the secreted antibodies), (ii) sort/isolate said B cells from other cells and (iii) to recover the viable B cells and/or the corresponding immunoglobulin variable heavy and light gene sequences.

In view of the above, there is a need to find improved or alternative methods for the identification of rare antibody-secreting cells producing an antibody with a binding and/or functional profile of interest. In particular, there is a need to provide a method enabling the identification of rare, low frequency, B cells expressing high quality antibodies meeting stringent requirements such as high biological activity or a particular multi-antigen binding profile.

### SUMMARY OF THE INVENTION

The current invention addresses some of these difficulties by providing an improved method for the identification and isolation of cells secreting antibodies of interest, e.g., having the desired biological activity and/or multi-antigen specificities. This method subsequently enables the retrieval of said cells and the cloning of said antibodies VH/VL sequences to generate recombinant monoclonal antibodies derived thereof having the features of interest.

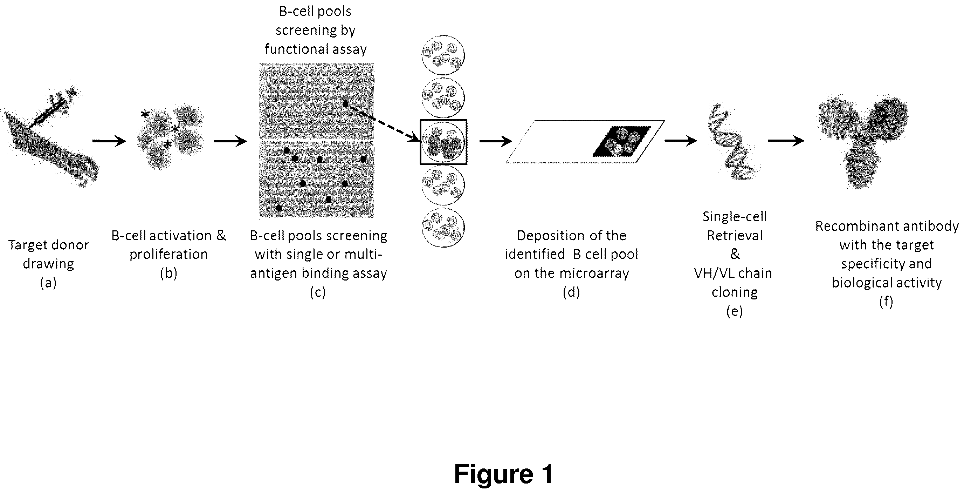

The method of the invention for the generation of new monoclonal antibodies with the desired biological activity and/or with particular binding profiles is schematically illustrated on **Figure 1**.

It was shown that by the early identification and selection of cell pools comprising B cells secreting antibodies meeting the pre-defined requirements of biological activity and/or binding profile to selected epitopes/antigens, the method of the invention provides a rapid and efficient method for the generation of monoclonal antibodies with the profile of choice by reducing the number of cells to be subsequently processed. In particular, as cell screening and subsequent cloning steps will only carried out on cells originating from those cell pools (hits) selected for meeting the requirements of biological activity/binding defined as objective of the antibody discovery campaign.

Furthermore, it was demonstrated that the method of the invention increases the recovery rate of the VH/VL gene pairs of a monoclonal antibody of interest, from corresponding antibody-secreting cells selected as a hit in the screening of antigen-specific antibody-secreting cells using an array, notably this is an advantage versus the ISAAC method described in Jin et al. (Nature Protocols, 2011, 6(5) 668-76).

Thus, in a first aspect, the present invention relates to a method for identifying, in a population of cells comprising antibody-secreting cells, a cell secreting an antibody against a target antigen said method comprising the steps of:
a) *in vitro* stimulation and amplification of the cells of said population and cell sampling in order to obtain multiple cell pools which contain expanded cell clones;
b) screening said cell pools supernatants to identify and select those cell pools containing cells secreting antibodies binding to said target antigen(s) and/or presenting a biological activity;
c) depositing the cells of said identified cell pools in an array comprising multiple wells in such conditions that at most three cells are present in each well of said array;
d) culturing the cells in the wells of said array and screening thereof to identify those wells containing a cell secreting an antibody against said target antigen(s).

In a second aspect, the invention provides a method for obtaining a cell producing a monoclonal antibody comprising the steps of:
a) isolating mRNA from a cell that is present in the identified wells of step d) of the method as described above, or from a cell culture obtained from one of said cells;
b) performing reverse transcription of said mRNA and amplifying the corresponding cDNA through polymerase chain reaction (RT-PCR);
c) cloning said DNA sequences corresponding to VH and VL regions in a suitable expression vector containing corresponding sequences coding for constant regions CH and CL, thus allowing expression of said VH and VL regions in the context of full length heavy and light chains.

It is to be noted that the VH and VL regions may be cloned from mRNA that has been isolated from different cells, harvested from different wells. Indeed, and as will be seen below, the array shall present multiple wells, each of which containing a cell secreting the antibody of interest. By design of the method of the invention (in particular the fact that the cells have been stimulated and thus expanded), said cells in the detected wells all originate from the same original cell by clonal expansion upon stimulation of step a).

In a another aspect, the present invention relates to a method for producing a monoclonal antibody, comprising the step of culturing a cell provided in the second aspect of the invention, or derived from said cell under such conditions that it expresses said monoclonal antibody.

In a further aspect, the present invention is concerned with the provision of_a method for the recovery of the VH and/or VL sequences of a monoclonal antibody comprising the steps of:
a) performing the method as described above and thus identifying, from a population of cells comprising antibody-secreting cells, a cell secreting an antibody against a target antigen; and
b) obtaining by RT-PCR the antibody VH and/or VL sequences from said identified cell, wherein
   i. the DNA of the VH and VL regions has been obtained from a cell isolated from a single well identified in step d) of the method as described above, or
   ii. the DNA of the VH region has been obtained from a cell isolated from a well identified in step d) of the method as described above and the DNA of the VL region has been isolated from a cell isolated from another well identified on the same array, in step d) of the method as described above, wherein, in step c) of the method as described above, all the cells that are present in a given array originate from the same cell pool.

The method according to the invention is performed on a population of cells comprising antibody-secreting cells that has been obtained from a biological sample that has been obtained from an individual prior to the implementation of the method. Consequently, the method according to the invention is performed *in vitro* and does not include any step performed on the individual.

### BRIEF DESCRIPTION OF THE FIGURES

**Figure 1****:** Schematic representation of the B cell screening method of the invention The screening method of the invention comprises the following steps: (a) Target donor or patient selection and drawing, followed preferably by (b) the activation and amplification of large populations of primary B cells from the donor, (c) the selection by high-throughput screening of B cell pools secreting antibodies with single or multi-antigen specificities and/or with biological activity. (d) the screening and detection of single B cells from the selected B cell pools in step c using single cell microarrays, (e) the retrieval of single B cells and the isolation of VH/VL gene pairs from each of the retrieved single B cells and (f) the production of recombinant monoclonal antibody candidates with the targeted single or multi-antigen specificity profile and/or biological activity.
**Figure 2**: Summary of 2 campaigns of fully human antibody discovery against gram positive bacteria toxin 2 with the method of the invention
**Figure 3****:** High efficiency cloning of functional hits by the method of the invention (campaign #1)
**Figure 4****:** Titration by ELISA of 6 purified recombinant monoclonal antibodies binding to bacterial toxin 2
   All 6 mAbs bind to bacterial toxin 2. Four of them are strongly binding to toxin 2 (mAb1, mAb2, mAb4, mAb5), while 2 of them are binding more weakly (mAb 7 and 8). A reference monoclonal antibody currently in late stage clinical development is used a positive control (Reference mAb).
**Figure 5****:** Titration by cytotoxicity assay of 6 purified recombinant monoclonal antibodies neutralizing bacterial toxin 2
   All 6 mAbs neutralize the toxin 2 cytotoxicity, and 3 of them were superior to a strong toxin 2 neutralizing reference antibody used as a positive control and currently in late stage clinical development (Reference mAb).
**Figure 6****:** High efficiency cloning of functional hits by the method of the invention (campaign #2)
**Figure 7****:** Summary of 2 ISAAC campaigns of discovery of fully human monoclonal antibodies against bacterial toxin 2
**Figure 8**: Comparison of binding and neutralizing activity of fully human monoclonal antibodies against bacterial toxin 2 obtained by ISAAC technology and the method of the invention
   Unpurified supernatants of CHO cells transfected with the expression vectors coding the H and L chain of the indicated antibodies were tested by ELISA for their binding to toxin 2 and by a functional assay for their neutralizing activity against toxin 2. The dilution of each sample was adapted in order to reach a final concentration of 0.3µg/mL human IgG in the assays. Negative control: supernatant of non-transfected CHO cells. Positive control: supernatant of CHO cells transfected with an expression vector coding the H and L chain of reference antibody against bacterial toxin 2 currently in clinical development (Reference mAb).

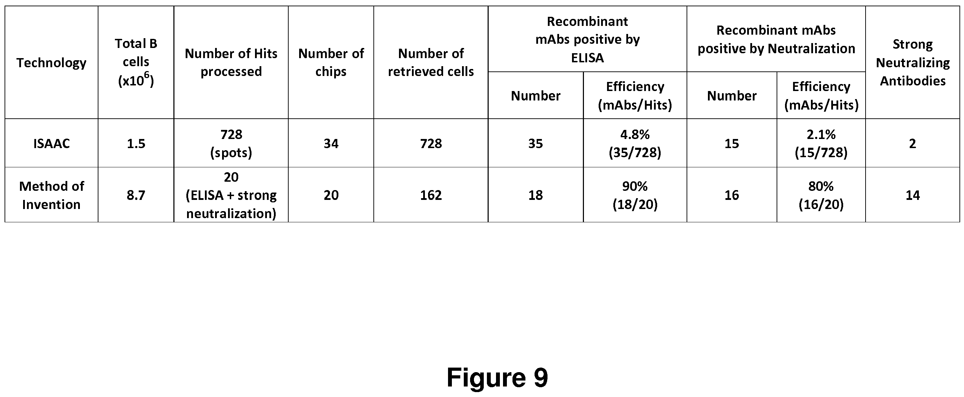

**Figure 9**: Comparison of the results of biologically active fully human monoclonal antibody discovery with the method of invention versus ISAAC technology for a comparable workload (10 weeks anti-toxin 2 campaigns with same donor)

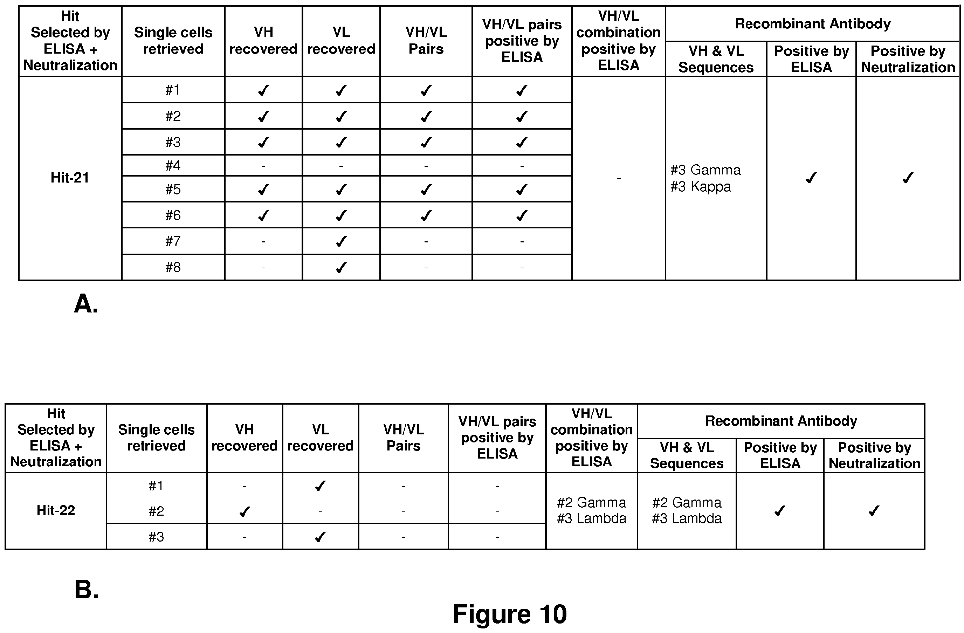

**Figure 10**: Examples of VH and VL gene recovery by VH and VL pairing or combination from single cells retrieved from the same B cell pool

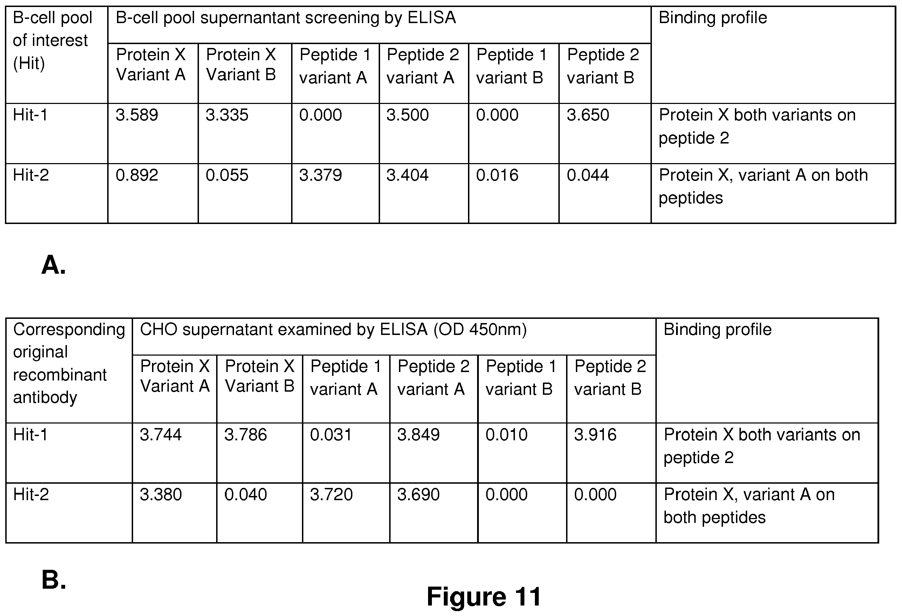

**Figure 11**: Generation of fully human monoclonal antibodies with targeted multi-antigen specificities

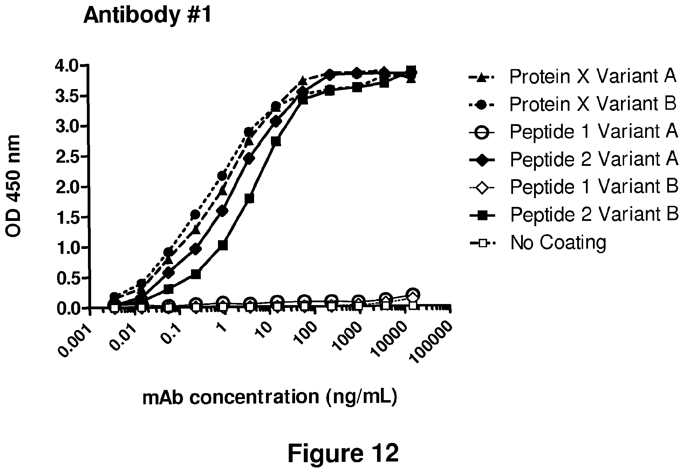

**Figure 12**: Purified recombinant monoclonal antibody #1 showing the target multi-antigen specificity obtained by the method of invention

### DETAILED DESCRIPTION OF THE INVENTION

### DEFINITIONS

### BIOLOGICAL SAMPLE

Cells that express and secrete antibodies are isolated from different tissues, organs and biological fluids from individuals. Cells may be isolated from the central or primary lymphoid organs that generate lymphocytes from immature progenitor cells, such as the thymus and the bone marrow. Alternatively, cells can be isolated from secondary lymphoid tissue that provides the environment for the antigen to interact with the lymphocytes, such as the lymph nodes, the lymphoid follicles in tonsils, Peyer's patches, spleen, adenoids, skin, etc. that are associated with the mucosa-associated lymphoid tissue (MALT).

It is noted that mononuclear cells in the spleen contain a higher percentage of IgG secreting cells. Cells can also be isolated from biological fluids such as blood, cerebrospinal or pleural fluids. Alternatively, tumor infiltrating lymphocytes, lymphocytes from sites of chronic infection and/or inflammation can be isolated.

The best sources of primary human B cells are splenic mononuclear cells, tonsils and peripheral blood mononuclear cells. (Olsson et al. J. Immunol. Methods 61 :17-32 (1983); Karpas A. Proc. Natl. Acad. Sci. USA 98:1799-1804 (2001)).

Peripheral blood is usually easier to obtain from donors, to store, and to monitor for the serological response against an antigen over a defined period of time. For example, starting from 5-50 ml of peripheral blood, approximately 10-100 million of PBMCs (peripheral blood mononuclear cells) can be purified, a number of cells that should allow obtaining a sufficiently large population of antibody-secreting cells to be screened after being immortalized using the methods of the Invention.

Biological samples, and specially blood, obtained from donors are usually screened for the presence of antibody having the ability to bind to the antigen of interest, and/or for the presence of functional antibody against an antigen of interest.

Cells of human origin are preferred for producing cell cultures secreting human monoclonal antibodies having therapeutic or diagnostic use, as fully human antibodies. Nonetheless, the methods may be applied on non-human, antibody-secreting cells such as cells of genetically modified, xenografted or wild type animals. For instance, these animal cells are from rodent (e.g., rats, mice, hamsters), avian (e.g., chicken, duck, goose, turkey), camel, rabbit, bovine, porcine, sheep, goat, or simian origin. Antibodies isolated from these sources may then be humanized, or be used in diagnostic methods.

### "INDIVIDUAL","DONOR", "PATIENT"

The cells that express and secrete antibodies are obtained from a human or non-human donor. A donor is an individual that donates a biological sample. Said individual can be human or non-human. A non-human donor can be a genetically modified, xenografted or wild type animal, such as rodent (e.g., rats, mice, hamsters), avian (e.g., chicken, duck, goose, turkey), camel, rabbit, bovine, porcine, sheep, goat or simian.

Fully human mAbs are increasingly recognized as superior therapeutic candidates over mouse-derived products, because the mAbs have been naturally selected and therefore have high affinity, are stable, and lack off-target reactivity against human antigens. Thus, preferably the donor will be a human.

Advantageously, the donor will be selected for presenting antibodies with the binding features of interest. For instance, the donor selection can be performed by carrying out an Enzyme Linked Immunosorbent Assay (ELISA) test or an equivalent assay to determine the presence of antibodies binding to one or more target antigens in a biological sample of the donor. Such methods are well known in the art.

The donor might be a "healthy", "untreated" or "naive" individual meaning that he has not been subject to an infection with an infectious agent or a chronic disease, or is not affected by a specific infectious agent or human disease for which antibodies are desired.

Alternatively, the donor has been subject to a common therapeutic or prophylactic vaccination; or where antibodies targeted to a specific infectious agent or human disease is desired.

A third alternative is that the donor is a "patient". Thus, the donor is selected for a population that suffers or has suffered from the disease, or has been infected by or vaccinated against a common infectious agent.

In one embodiment, the patient has been subject to/suffered from a specific disease and has recovered from said disease, in particular due to humoral response. In another embodiment, the patient has been exposed to an infectious agent and not developed the disease or developed a mild version of the same.

In a preferred embodiment, said patient has been demonstrated to have an antibody with a functional activity against a disease linked to said target antigen, for instance, a neutralizing antibody response against an infectious agent.

In one preferred embodiment, the donor is a patient that has suffered/developed at least one disease, selected in the group of:
- Infectious disorders: such as influenza viral infection, hepatitis C virus (HCV) infection, hepatitis B virus (HBV) infection, herpes simplex virus (HSV) infection, human immunodeficiency virus (HIV) infection, Chikungunya virus infection, rabies, other hepatitis viruses such as hepatitis A or C viruses infections, Cytomegalovirus (CMV) infection, *Staphylococcus aureus* infection, Methicillin-resistant *Staphylococcus aureus* (MRSA) infection, *Staphylococcus non-aureus* infection, *Streptococcus pneumoniae* infection, Epstein-Barr virus (EBV) infection, respiratory syncytial virus (RSV) infection, Pseudomonas infection such as *Pseudomonas aeruginosa* infection, Candida infections; *Clostridium difficile* infection, *Propionibacterium acnes* infection, *Porphyromonas gingivalis* infection, Coagulase-negative staphylococci (CoNS) infection, *Klebsiella pneumoniae* infection, *Escherichia coli* infection and in particular enterohemorrhagic *E coli* infections, *Acinetobacter* infection, *Leptospira patoc* infection or *Chlamydiae.* Infection.
- Respiratory disorders such as asthma, allergies, chronic obstructive pulmonary disease (COPD), idiopathic pulmonary fibrosis (IPF), adult respiratory distress syndrome (ARDS);
- Metabolic disorders such as frailty, cachexia, sarcopenia, obesity, dyslipidemia, metabolic syndrome, myocardial infarction (MI), chronic renal failure (CRF), osteoporosis
- Digestive disorders such as irritable bowel syndrome (IBS), inflammatory bowel disease (IBD), Crohn's disease, fatty liver disease, fibrosis, drug-induced liver disease;
- Neurological disorders such as Alzheimer's disease, multiple sclerosis (MS), Parkinson's disease, bovine spongiform encephalopathy (BSE, mad cow disease);
- Cancers such as breast, renal, stomach, melanoma, pancreas, lung, colon, glioma, glioblastoma, lymphoma, leukemia and prostate cancer;
- Autoimmune diseases, such as rheumatoid arthritis, autoimmune cardiomyopathy, autoimmune hemolytic anemia, autoimmune hepatitis autoimmune lymphoproliferative syndrome and other autoimmune diseases.

### TARGET ANTIGEN(S)

The method of the invention may be used against self antigens, such as Interleukin 1 alpha, Interleukin 17, Platelet GPIa/Ilb and antigens from the extracellular part of transmembrane proteins, intracellular proteins or full transmembrane proteins such as growth factor receptors.

The nature of the target antigen is not specifically limited; various desired members of the group consisting of proteins, sugars, lipids, nucleic acids, organic compounds, inorganic compounds, and combinations thereof (including cells) can be suitably selected.

According to a preferred embodiment, the antigen used for the selection of antibody of interest is soluble (e.g bacterial toxin, viral protein, cytokine, etc...). According to a preferred embodiment, the antigen used for the selection of antibody of interest is an antigen in its native conformation.

### BINDING SUBSTANCE

This binding substance has the ability to bind to at least a portion of an immunoglobulin of interest. The binding substance may thus be the target antigen or an anti-immunoglobulin antibody capable of reacting against immunoglobulin (such as a anti-IgG antibody). Type of usable binding substances will be later described. It is not excluded that the binding substance is modified (grafting of a moiety) in order to allow detection of binding of the antibody of interest.

### LABEL SUBSTANCE

A label substance makes it possible to detect production of the antibody of interest.

The label substances are generally proteins or small molecules that have been modified in order to be cross-linked with a detectable moiety, in particular a fluorescent moiety, or have been designed to be detectable (i.e. fluorescent) by themselves. However, it is not limited to fluorescent agents, and other kind of detectable labels (such as radiolabeled moieties) are possible. Labelling of a protein such as an antigen or antibody with a fluorescent moiety can be conducted by usual methods (such as the ones that are used for labelling proteins for ELISA detection).

The label substance can either bind to the antibody of interest or to the binding substance, as is further described. The label substance contains a moiety that makes possible to detect presence or absence of said binding substance. This moiety may be a fluorescent (for direct detection or detection by FRET if the binding substance is adequately labelled), or a radio-labelled moiety.

The invention can be performed to simultaneously detect antibody-secreting cells expressing antibodies binding to different antigens (to be clear, the antibody from a specific antibody-secreting cell shall only bind to a specific antigen, or to closely related (with regards to epitopes antigens). In that case, one shall use the different target antigens as label substances, each of which being differently labelled so that it is possible to selectively identify the cells expressing the antibodies against each target antigen (i.e multiplex antibody screening). This can also permit identification of cells expressing antibodies reacting to the closely related target antigens.

### ANTIBODY

The term "antibody" as used herein refers to monoclonal antibodies, fragments thereof, and immunologic binding equivalents thereof. A monoclonal antibody is capable of selectively binding to a target antigen or epitope. The term "antibody" broadly encompasses naturally-occurring forms of antibodies (for example, IgD, IgG, IgA, IgM, IgE) and recombinant antibodies such as single-chain antibodies, chimeric and humanized antibodies and multi-specific antibodies. The term "antibody" also refers to fragments and derivatives of all of the foregoing, and may further comprise any modified or derived variants thereof that retain the ability to specifically bind an epitope. Antibody derivatives may comprise a protein or chemical moiety conjugated to an antibody. Antibodies may include, but are not limited to monoclonal antibodies (mAbs), humanized or chimeric antibodies, camelized antibodies, single chain antibodies (scFvs), Fab fragments, F(ab')₂ fragments, disulfide-linked Fvs (sdFv) fragments, anti-idiotypic (anti-Id) antibodies, intra-bodies, synthetic antibodies, and epitope-binding fragments of any of the above. The term "antibody" also refers to fusion protein that includes a region equivalent to the Fc region of an immunoglobulin. It should be noted, however, as it would be evident to a person skilled in the art that the antibodies secreted by an antibody-producing cell originating from a biological sample from a donor will be naturally-occurring forms of antibodies.

### VECTOR/EXPRESSION VECTOR

Vectors may be autonomously replicating or may replicate together with the chromosome into which they have been integrated. The nucleic acid molecule can be operably linked to a control sequence. Furthermore, the vector may additionally contain a replication origin or a selection marker gene.

Preferably, said vectors are used in eukaryotic cells, preferably, in mammalian cells. Expression vectors used in eukaryotic host cells (yeast, fungi, insect, plant, animal, human, or nucleated cells from other multicellular organisms) typically also contain sequences necessary for the termination of transcription and for stabilizing the mRNA. Such sequences are commonly available from the 5' and, occasionally 3', untranslated regions of eukaryotic or viral DNAs or cDNAs. One useful transcription termination component is the bovine growth hormone polyadenylation region. See for example, WO 94/11026 and the expression vector disclosed therein.

### HOST CELL

Host cells include, but are not limited to, cells of mammalian, plant, insect, fungal or bacterial origin. Transformation could be done by any known method for introducing polynucleotides into a host cell, including for example packaging the polynucleotide in a virus (or into a viral vector) and transducing a host cell with the virus (or vector) or by transfection procedures known in the art, as exemplified by US 4,399,216, US 4,912,040, US 4,740,461, and US 4,959, 455.

Particularly, methods for introducing heterologous polynucleotides into host cells are well known in the art and include dextran-mediated transfection, calcium phosphate precipitation, polybrene mediated transfection, protoplast fusion, electroporation, encapsulation of the polynucleotide(s) in liposomes, and direct microinjection of the DNA into nuclei.

Examples of host cells that may be used according to the present invention include but are not limited to eukaryotic cells such as mammalian cells, e.g. hamster, rabbit, rat, pig, mouse, etc.; avian cells, e.g. duck, chicken, quail, etc.; insect cells or other animal cells; plant cells and fungal cells, e.g. corn, tobacco, Saccharomyces cerevisiae, Pichia pastoris; prokaryotic cells such as E. coli; and other cells used in the art for the production of antibodies and other binding proteins. Especially mammalian cell lines available as hosts for expression are well known in the art and include many immortalized cell lines available from the American Type Culture Collection (ATCC), including but not limited to Chinese hamster ovary (CHO) cells, HeLa cells, baby hamster kidney (BHK) cells, monkey kidney cells (COS), human hepatocellular carcinoma cells (e.g. Hep G2), myeloma cells (e.g Sp2/0, NS0, YB2/0) and a number of other cell lines. Preferably, the host cells are human cells. Examples of human cells are inter alia HeLa, 911, AT1080, A549, 293, HEK293T and Per.C6® cells. In another preferred embodiment, the host cells are non-immortalized cell lines.

Alternatively said host cell is an avian cell. Avian cell lines may be derived from a variety of developmental stages including embryonic, chick and adult. Avian cell lines may be genetically modified or not. Preferably, the cell lines are derived from the embryonic cells, such as embryonic stem cells, embryonic fibroblasts, germ cells, or individual organs, including neuronal, brain, retina, kidney, liver, heart, muscle, or extra-embryonic tissues and membranes protecting the embryo. Examples of avian cell lines include avian embryonic stem cells (WO01/85938), immortalized duck retina cells (WO2005/042728), and genetically modified avian cells expressing telomerase reverse transcriptase (WO2007/077256 and WO2009/004016). Suitable avian embryonic derived stem cells, include the EBx cell lines derived from chicken embryonic stem cells such as EB45, EB14 and EB14-074 (WO03/07660 and WO2006/108846) or derived from duck embryonic stem cells, such as EB66, EB26, EB24 (WO2008/129058 and WO2008/142124). More preferably, the duck cell line is EB66 cell line.

### CULTURE MEDIUM

By "cell growth medium", "cell culture medium" or "culture media" or "media formulation" it is meant a nutritive solution for culturing or growing cells. The ingredients that compose such media may vary depending on the type of cell to be cultured. In addition to nutrient composition, osmolarity and pH are considered important parameters of culture media.

The cell growth medium comprises a number of ingredients well known by the man skilled in the art, including amino acids, vitamins, organic and inorganic salts, sources of carbohydrate, lipids, trace elements (CuSO₄, FeSO₄, Fe(NO₃)₃, ZnSO₄...), each ingredient being present in an amount which supports the cultivation of a cell *in vitro* (i.e survival and growth of cells). Ingredients may also include different auxiliary substances, such as buffer substances (like sodium bicarbonate, Hepes, Tris...), oxidation stabilizers, stabilizers to counteract mechanical stress, protease inhibitors, animal growth factors, plant hydrolyzates, anti-clumping agents, anti-foaming agents. If required, a non-ionic surfactant, such as polypropylene glycol can be added to the cell growth medium as an anti-foaming agent.

The cell growth medium is preferably an animal serum-free medium" (SFM), which meant that the cell growth medium is ready to use, that is to say that it does not required serum addition allowing cells survival and cell growth. The cell growth medium is preferably chemically defined, but it may also contained hydrolyzates of various origin, from plant for instance. Preferably, said cell growth medium is "non animal origin" qualified, that is to say that it does not contain components of animal or human origin (FAO status: "free of animal origin"). In SFM, the native serum proteins are replaced by recombinant proteins. Alternatively SFM medium according to the invention does not contain protein (PF medium: "protein free medium") and/or are chemically defined (CDM medium: "chemically defined medium"). It is well known in the art that the cell growth medium may be supplemented with defined supplements such as antibiotic to prevent bacterial contamination. An example of antibiotics includes gentamycin, penicillin and streptomycin. Gentamycin is usually used at a final concentration of 10 ng/ml, penicillin at a final concentration of 100 U/ml and streptomycin at a final concentration of 100 µg/ml. The cell growth medium may also be supplemented at different moments during cell growth with defined supplements such as vitamins, sugars or amino acids.

### PROTEIN VARIANT

The term "variant" as used herein encompasses the terms "modified", "mutated", "polymorphisms" or "mutant", terms which are interchangeable. The term "variant" refers to a gene product (i.e., antigen or target protein) which displays modifications in sequence and or functional properties (i.e., altered characteristics) when compared to the gene product of reference (i.e., antigen or target protein); usually, the gene product of reference is the wild-type gene product. The term "wild-type" refers to a gene product which has the characteristics of that gene product when isolated from a naturally occurring source. A wild-type gene is that which is most frequently observed in a population and is thus arbitrarily designed the "normal" or "wild-type" form of the gene. It is noted that naturally-occurring variants can be isolated; these are identified by the fact that they have altered characteristics when compared to the wild-type gene product.

In a first aspect, the present invention relates to a method for identifying a cell secreting an antibody against a target antigen, said method comprising the steps of:
a) *in vitro* stimulation and amplification of the cells of said population and cell sampling in order to obtain multiple cell pools which contain expanded cell clones;
b) screening said cell pools supernatants to identify and select those cell pools containing cells secreting antibodies binding the target antigen(s) and/or presenting a biological activity;
c) depositing the cells of said identified cell pools in an array comprising multiple wells in such conditions that, statistically, at most three cells are present in each well of said array;
d) culturing the cells in the wells of said array and screening thereof to identify those wells containing a cell secreting an antibody against the target antigen(s).

It is to be noted that the term "a" is to be interpreted as meaning "one or more". Thus, said method also makes it possible to identify cells secreting antibodies against more than one antigen, as well as against one specific antigen. The cell pool supernatants are optionally recovered from the cell pools container, e.g., well in a 96-well plate.

The stimulation of the antibody-secreting cells population in step a) and the cell sampling of step b) can be in any order. Thus, one can first stimulate the cells (which would lead to amplification) and then sample the amplified cells. This alternative is preferred.

As an alternative, one can sample the cells first and then stimulate them in order to lead to amplification of the cells.

In any case, one will obtain, after step a) multiple pools of cells, which contain of expanded cell clones.

In a preferred embodiment, when performing step c), the cells of each of said identified cell pools are deposited in at least one array in such a way that all the cells in an array correspond to the same cell pool.

In a particular embodiment, the invention relates to a method for identifying, in a population of cells comprising antibody-secreting cells, a cell secreting an antibody presenting biological activity against one or more target antigens, comprising the steps of:
a)
   a1. Stimulating the cells in said population of cells with at least one stimulating agent in order to obtain stimulated antibody-secreting cells in which antibody secretion is initiated and/or increased and sampling said stimulated antibody secreting cells in order to obtain multiple cell pools; or
   a2. Sampling said population of cells in order to obtain multiple cell pools and stimulating the cells in each of said pools with at least one stimulating agent in order to initiate and/or increase antibody secretion from antibody-secreting cells present in the pools
b) Contacting a sample of culture medium from each pool with said target antigens in order to identify culture medium samples containing at least one antibody having the desired functional activity and/or antigen-binding specificity, thereby identifying pools containing at least one cell secreting an antibody presenting biological activity against said target antigen and/or identifying pools containing at least one cell secreting an antibody presenting binding activity against said target antigen, and further
c) Depositing said cells from said identified pools on an array comprising multiple wells, in such conditions that, statistically, at most three cells are present in each well of said array;
d) Culturing the cells in the wells of said array and contacting supernatant of each well in said array with said target antigen thereby identifying wells containing a cell secreting an antibody against said target antigen where said target antigen binds to antibody present in said supernatant binds to said target antigen.

### SELECTION OF POPULATION OF CELLS THAT SECRETE ANTIBODIES

Mononuclear Cells (MC) are isolated from the biological sample by methods known in the art. Preferably, the donor from which said biological sample originates has been previously screened positive for the presence of immunoglobulins binding to the antigen of interest it its serum, as above described. For blood samples, Peripheral Blood Mononuclear Cells (PBMCs) are typically isolated using Ficoll® gradient separation by centrifugation.

After the isolation of PBMCs from the biological samples, a specific selection of antibody-secreting cells can be performed, using one of the many methods described in the literature, on the basis of the expression of specific antibody isotypes and/or cell surface markers on their surface and, if appropriate, of other proteins, as well as the proliferation activity, the metabolic and/or morphological status of the cells.

In particular, various technologies for the purification of antibody-secreting cells from human samples make use of different means and conditions for positive or negative selection. These cells are more easily and efficiently selected by physically separating those expressing cell surface markers specific for cells that express and secrete antibodies (e.g. human B cells). Specific protocols can be found in the literature (see Callard R and Kotowicz K "Human B-cell responses to cytokines" in Cytokine Cell Biology: A practical Approach. Balkwill F. (ed.) Oxford University Press, 2000, pg.17-31).

In a specific embodiment, the population of cells comprising antibody-secreting cells is a subpopulation from a blood sample that has been obtained by positive or negative selection of antibody-secreting cells by using particular markers in the cell surface. For instance, lymphocyte B cells might be selected by depletion of other PBMCs such as T lymphocytes (i.e. CD2+ cells) or for the presence of specific surface markers, such as CD20, CD19, CD27 and/or CD22 as provided below. Said subpopulation might also be selected on the basis of the ability for the cells to express antibodies of specific isotypes (such as IgG, IgM, IgA, IgE or IgD). It is often preferred to select a subpopulation of IgG-secreting cells. In another embodiment, the subpopulation may be depleted in cells secreting certain isotypes that might interfere with the screening process and/or limit the efficacy of the downstream process. In a preferred embodiment said cell population is enriched in IgG-secreting cells by depletion from IgM-expressing and/or IgD-expressing cells. Preferably, said cell population is depleted from IgM-expressing and IgD-expressing cells.

Advantageously, the antibody-secreting cells population is selected on the basis of the expression of at least a cell surface marker. Accordingly, the selection is usually performed using antibodies that bind specifically to one of these cell surface proteins and that can be linked to solid supports (e.g. microbeads or plastic plates) or labeled with a fluorochrome that can be detected flow cytometry. For example, human B cells have been selected on the basis of their affinity for supports (such as micro-beads) binding CD20, CD19, CD27 and/or CD22 antibodies, or for the lack of binding affinity for antibodies specific for certain isotypes. Preferably, this selection step is performed prior to the transformation with a lymphotropic virus, e.g. EBV immortalization (Traggiai E et al., Nat Med. 2004 Aug;10(8):871-5. Epub 2004 Jul 11).

However, the choice of the cell marker may be relevant for the efficiency of the immortalization process, probably due to intracellular signals that are triggered by the selection process and that may alter cell growth and viability. CD22, which is a B-cell restricted transmembrane protein that controls signal transduction pathways related to antigen recognition and B cell activation (Nitschke L, Curr Opin Immunol. 2005 Jun;17(3):290-7), appears as a preferred molecule for the initial B cell selection. Since the CD22 positive population contains cells that express antibodies having different isotypes and specificities, other cell surface markers can be used for selecting the cells, either before or after the stimulation phase.

Alternatively or additionally, a specific enrichment of antibody-secreting cells can be obtained by applying a CD27-based selection in addition to the CD22-based selection. CD27 is known to be a marker preferentially expressed by human memory B cells that have somatically mutated variable region genes (Borst J et al., Curr Opin Immunol. 2005 Jun;17(3):275-81). Additional markers such as CD5, CD24, CD25, CD86, CD38, CD45, CD70, CD158 or CD69 could be used to either deplete or enrich for the desired population of cells. Thus, depending on the donor's history of exposure to the antigen (e.g. viral, bacterial, parasite), the antibody titer, a decision can be taken as to whether to use total, CD22 enriched B cells, or further enriched B cell subpopulations such as CD27 positive B cells. Consequently, in a preferred embodiment, in particular, the selected cell population consists in B lymphocytes, comprising at least one cell surface marker selected in the group consisting of CD20, CD22, CD19, and CD27.

### ISOTYPE SELECTION

Antibody secreting cells can be selected on the basis of the isotype of the expressed antibody before or after stimulating the cells. Preferably, said selection step is carried out prior to the antibody-secreting cells population stimulation and amplification of step a).

The isotype-based selection of the cells should be performed by applying means for either positive selection (allowing the isolation of the specific cells) or negative selection or enrichment (allowing the elimination of unwanted cells) selection. For example, given that most therapeutic antibodies approved for pharmaceutical use are IgG (Laffy E and Sodoyer R, Hum Antibodies. 2005;14(1-2):33-55), only a population of stimulated IgG positive cells can be selected positively (by FACS or magnetic cell separators) or by depleting cells that express other isotypes such as IgM from the population of cells, and consequently enriching for cells that express IgG. According to a preferred embodiment, the population of cells comprising antibody-secreting cells is a subpopulation of cells that has been depleted in cells that express IgM and/or IgD, preferably depleted of both IgM and IgD expressing cells. The IgM depletion is preferably performed before stimulation. According to another embodiment, when immortalization is performed before stimulation, the IgM depletion is preferably performed before immortalization. Alternatively, IgM depletion is performed between stimulation (e.g CpG-2006 and Interleukin 2) and immortalization (e.g., with Epstein Barr Virus) as described in WO2007/068758.

Separation technologies for antibody-secreting cells using fluorescence activated or magnetic cell separators are known in the literature (Traggiai E et al., op.cit.). Depending on the source of antibody-secreting cells and their final use, depletion (or enrichment) of IgD, IgM, IgE or IgA expressing cells may also be desired.

A similar approach can be used for isolating cells on the basis of the specific subclass, if such a precise selection is desired (e.g., distinguishing human B cells that express IgG1, IgG2, IgG3, or IgG4 antibodies).

### IMMORTALIZATION OF CELLS

In a specific embodiment the antibody-secreting cells (B cells) are transformed/immortalized before, during or after step a).

It is to be noted that a viral transforming agent might trigger a cellular process leading to the cell indefinite self-renewal, i.e., to the cell immortalization. The use of the term "transformed" or "immortalized" is a time-related question. Accordingly, in the present invention the terms "transformation" and "immortalization" and derivatives thereof, will have the same meaning and can be used equally. In particular, this represents the fact that the antibody-secreting cells are still alive after stimulation and proliferation.

As mentioned below, said transformation is preferably carried out using a transforming viral agent, more preferably the antibody-secreting cells are infected with a lymphotropic virus before or during step a). In a preferred embodiment, said immortalization is performed with Epstein-Barr virus.

Different techniques may be used to immortalize B cells.
1) Fusion of B cells with myeloma cells of murine, human and murine x human origin to generate hybridoma;
   WO2009/105150: CD27+ B cells cultured in presence of II-4, II-10 and CD40L can be fused with a fusion partner to generate human B cell hydridomas. The advantage of using treated CD27+ B cells in the generation of hybridomas is that a higher percentage of cells in the hydridoma secreted IgG antibodies.
2) Viral transformation of B cells with Epstein-Barr Virus (EBV) (as described below)
3) Transduction of B cells with lentiviral vectors expressing transforming gene, such as described in US 4,997,764 or US5,798,230

### Viral transformation

According to a preferred embodiment, the selected and stimulated population of cells that express antibodies may be immortalized using a viral immortalizing agent. Literature shows that different immortalizing agents can be used on antibody-secreting cells, and sometimes even combined in a single process in order to obtain immortalized antibody-secreting cells.

Amongst the viral immortalizing agents, a virus that infects and immortalizes antibody-secreting cells should be preferably used in the methods of the invention. Viruses having such preference are commonly known as lymphotropic viruses and are grouped in the gamma class of herpesvirus.

Members of this virus family infect lymphocytes in a species-specific manner, and are associated with lymphoproliferative disorders and the development of several malignancies. EBV (Epstein-Barr virus, also known as herpesvirus 4), and HHV-8 (human herpesvirus 8, also known as KSHV, Kaposi's Sarcoma associated Herpervirus) infect and immortalize human lymphocytes. MHV-68 (murine herpesvirus 68), HVS (herpesvirus Samiri), RRV (Rhesus Rhadinovirus), LCV (primate Lymphocrypto virus), EHV-2 (Equine Herpesvirus 2) HVA (Herpesvirus Ateles), and AHV-I (Alcelaphine Herpesvirus 1) are other oncogenic, lymphotropic herpesvirus having some common genetic features conserved amongst them and similar pathogenic effects in different mammalian host cells. These viruses can be used whenever the methods of the Invention are applied on antibody-secreting cells obtained from such mammals.

However, not only full viruses can immortalize B cells since recombinant DNA constructs that contains specific viral proteins obtained by such specific virus and other virus have been successfully used to immortalize B cells (Damania B Adv Cancer Res. 2001;80:51-82; Kilger E et al., EMBO J. 1998 Mar 16;17(6):1700-9). Similar vectors containing viral genes can be transduced into cells, sometimes making use of retroviral systems or virus-like particles into packaging cell lines which provide all the necessary factors in trans for the formation of such particles, can also be used in the methods of the invention.

The immortalization phase can last between 1 and several hours, up to 2-4 days, in the case of EBV at least, 4 hours can be sufficient to establish polyclonal populations of lymphoblasts (large viable cells, as measured by microscopy and or FACS) that provide immortalized antibody-secreting cells.

EBV-mediated immortalization of B cells is performed in presence of the cell surface receptor CD21 which is considered as the main EBV receptor. CD21 is present on most B cell subpopulations and regulates B cell responses by forming a complex with CD19 and the B cell antigen receptor (Fearon D and Carroll M, Annu Rev Immunol. 2000;18:393-422). However, CD21 is lost from the cell surface following extensive activation of cells, and as they transform into plasma cells. Thus, the ability to transform cells with EBV may be aided by the addition of B cell stimulating agents, but the conditions should preferably ensure that CD21 is maintained on the cell surface, allowing EBV immortalization at high efficiency.

Immortalized populations of antibody- secreting cells can be efficiently obtained. In fact, cell culture populations enriched for B cells that are selected and immortalized have a greater likelihood to produce useful therapeutic antibodies, while maintaining their ability to grow when immortalized with EBV virus in a latent, and not lytic, state. The process of immortalization allows the population to be "captured" in a state of high proliferative and IgG-secreting capacity.

EBV-mediated immortalization is a complex process involving the immortalization of B cells due to proteins that are expressed by EBV, followed by the immortalization regulated by the interaction between EBV and host cells proteins (Bishop GA and Busch LK, Microbes Infect. 2002 Jul;4(8):853-7).

The amount of EBV supernatant to be added to the cell culture can be that commonly indicated in the literature (10%, 20%, 30%, or more), but it appears that the methods can work properly in conditions in which the amount of EBV supernatant is relatively high (50% V/V) but the exposure is relatively short (from about 4 to about 24 hours).

Optionally but preferably, the viral immortalizing agent is eliminated, for example by washing and culturing the population of cells, into fresh cell culture medium.

It is to be noted that *in vitro* B-cell immortalization with EBV can also be promoted by oxidative stress such as the one induced by cyclosporine A and hydrogen peroxide, (used at concentrations of around 500 ng/ml and around 100 µM, respectively).

### STIMULATION OF CELLS

The purpose of this step is to have the cells to secrete antibody in the culture medium. As indicated above, the cells that can produce the antibody of interest may be memory B cells and/or any antibody-secreting cells and thus need to be stimulated in order to efficiently secrete the antibody, such that there is a detectable amount. Stimulation will lead to clonal expansion of the cells of the population. This stimulation might be specific by using the target antigen as stimulating agent but is preferably non-specific (ie, all antibody-secreting cells are stimulated).

This is also a purpose of this step to maintain antibody-secreting cells viable long enough to, or preferably to induce proliferation of the cells in the culture medium for a time necessary and sufficient to, detect the memory B cells and/or the antibody-secreting cells expressing antibody binding to the antigen of interest and/or functionally active during the step of sampling the cells below described.

In the present invention, the terms "stimulation" and "activation" will have the same meaning and can be used equally.

Stimulating agents can be chosen amongst the following compounds:
a) At least one agonist of Toll Like Receptor (TLR) which is expressed by B cells. Among the different TLRs expressed, TLR9 and TLR7 are preferred. TLR9 recognizes oligonucleotides, and more specifically CpG-based oligonucleotides. TLR7 recognizes single-stranded RNA, guanosine analogs and imidazoquinoline compounds such as imiquimod and resiquimod (R848).According to a preferred embodiment, the stimulating agent is a CpG-based oligonucleotides, and more preferably CpG2006 for human B cells, or is R848.
b) At least one cytokine known to have immune-stimulating activities such as Interleukin 2 (IL-2), Interleukin 4 (IL-4), Interleukin 6 (IL-6), Interleukin 10 (IL-10), Interleukin 13 (IL-13), Interleukin 21 (IL-21)
   According to a preferred embodiment, the stimulation of B cells is performed by combining stimulation with a CpG-based oligonucleotide or R848 (stimulating TLR9 or TLR7 respectively) and a cytokine (such as IL-2, IL-4, IL-10, IL21, IFN-gamma and the like), and more preferably by combining CpG2006 and IL-2.
c) At least an agonist of cell membrane receptors of the TNF receptor family, in particular those activating NF-KB pathway and proliferation in B cells, such as APRIL, BAFF or CD40L. According to a preferred embodiment, the B cells are co-stimulated by cross-linking B cell surface CD40 using anti-CD40 monoclonal antibody decorating the surface of mouse L cells transformed stably to express the Fc receptor for the anti-CD40 monoclonal antibody (EP0505397). According to another preferred embodiment, the B cells are co-stimulated by cross-linking B cell surface CD40 using CD40 ligand decorating the surface of mouse L cells. The use of soluble CD40 Ligand or agonistic antibodies against CD40 has been reported (WO 91/09115; WO 94/24164; Tsuchiyama L et al., Hum Antibodies. 1997;8(1):43-7; Imadome K et al., Proc Natl Acad Sci USA. 2003 Jun 24;100(13):7836-40. Epub 2003 Jun 12).

According to another preferred embodiment, the stimulation of B cells is performed by combining of an agonist of a cell membrane receptor of the TNF receptor family and a cytokine.

Other known stimulating agents are PWM (pokeweed mitogen), LPS (Lipopolysaccharide) or Staphylococcus aureus Cowan (SAC).

The combination of stimulating agents can be added to the cell culture medium before the immortalization phase if any, at the same time or sequentially (e.g. adding a first stimulating agent immediately after the initial cell selection and a second stimulating agent hours or days later), or after the immortalization phase, if this proves to be useful to obtain a better response from the antibody-secreting cells.

The cells are preferably cultivated in presence of an agent capable of cross-linking its CD40 antigen. The CD40 cross-linking stimulation is particularly preferred when EBV immortalization is carried out. Preferably, the cross-linking agent is an immobilized monoclonal antibody specific for CD40. It can be immobilized on a solid phase or non-aqueous phase liquid substrate, such as microspheres, liposomes, or cellular membranes. In addition the culture growth rate may be enhanced by the presence of the cytokines interleukin-2 (IL-2), interleukin-4 (IL-4), interleukin-6 (IL-6), interleukine-21 (IL-21) and interferon-gamma (INF- gamma), either alone or in combination. A detailed description of the CD40-specific antibodies and of substrates to be used to immobilize said antibody can be found in EP 505397. Alternatively, the B cells are co-stimulated by cross-linking B cell surface CD40 using CD40 ligand decorating the surface of mouse L cells (i.e., CD40L expressing feeder cells).

Infecting the cells with Epstein Barr virus (EBV) makes it possible to immortalize them (James, Scand. J. Immunol., Vol. 29, pg. 257; 1989). As indicated in EP 505397, the presence of a CD40 cross-linking agent increases the efficiency of B cell infection by EBV and hence the number of cells that are immortalized. Usually, the B cells are EBV-immortalized first, then 30 minutes later, 1 h later, 2h later, 3h later, etc...up to 1 day, 2 day, 3 day later, the B cells are stimulated with a CD40 cross-linking agent.

When EBV immortalization is performed, the cells are preferably cultivated in presence of a combination of CpG-based oligonucleotides, more preferably CpG2006, and a cytokine, more preferably IL-2. On the basis of its stimulatory properties, CpG2006 and IL-2 has been used simultaneously with EBV for producing immortalized human B cells (Traggiai et al., 2004; WO 04/76677) or before EBV immortalization (WO2007/068758),

The stimulating agents can be directly added in the cell culture medium from diluted stock solutions, or after being appropriately formulated, for example using liposomes or other compounds that can improve their uptake and immune-stimulatory activity (Gursel I et al., 2001). The stimulating agents may also be attached to solid matrices (microbeads or directly on the cell culture plates) also allowing a more effective removal.

After stimulation, and given the nature of the stimulants, the antibody-secreting cells are then preferably manipulated in a way that the stimulating agent is efficiently eliminated, in order to avoid any negative effect on the later immortalization and maintenance in cell culture conditions.

Thus, cells can be washed with fresh medium one or more times and, optionally, maintained in normal cell culture medium (for example, from 1 up to 6 days) in order to further dilute and eliminate any remaining effect of the stimulating agents, which may be even inhibited by adding specific compounds into cell culture.

### SAMPLING OF CELLS

As indicated above, it is frequent that the cells secreting the antibodies of interest are in very low number with regards to the total number of cells present in the biological sample.

Consequently, the method according to the invention comprises the step of sampling/dividing the cells in order to obtain multiple cell pools. In a preferred embodiment, each cell pool to be used in step b) contains between 10,000 and 1,000,000 antibody-secreting cells, between 50,000 and 900,000 antibody-secreting cells, between 80,000 and 600,000 antibody-secreting cells between 100,000 and 800,000 antibody-secreting cells. In a preferred embodiment, each cell pool contains 500,000 antibody-secreting cells +/- 10 %, more preferably around 100,000 to 300,000 cells.

It is to be noted that the cells can be sampled after stimulation. In this case, the pools contain 10,000 and 1,000,000 antibody-secreting cells, between 100,000 and 800,000 antibody-secreting cells, between 200,000 and 700,000 antibody-secreting cells, between 300,000 and 600,000 antibody-secreting cells. In the most preferred embodiment, each cell pool contains 500,000 antibody-secreting cells +/-10 %. In this case, the culture medium of the cells may be changed after sampling in order to avoid that it contains some antibodies that would have been produced by cells that are not represented in the pool.

If stimulation is performed after sampling, the pools preferably contain between 10 and 5000 antibody-secreting cells, between 20 and 2000 antibody-secreting cells, between 50 and 1000 antibody-secreting cells, between 50 and 500 antibody-secreting cells. In the most preferred embodiment, each cell pool contains 100 antibody-secreting cells +/- 10 %. Stimulation/activation may lead to clonal expansion of the antibody-secreting cells, thereby increasing the number of cells in the pool prior to step b).

The total number of cells introduced in each pool will depend on the percentage of antibody-secreting cells within the cell population.

Generally, the different pools are in wells of a microtiter plaque, usually 96 or 384 well-plaques.

Preferably, after cell sampling, said cell pools are cultured in a culture medium under conditions in which cells secrete antibodies into said culture medium, such as for example at 37°C in a 5% CO₂ atmosphere. Appropriate culture cell media have been described above. Consequently, some of the stimulating agents as mentioned above may be present in the culture medium. The goal of this operation is to make sure that the culture medium contains enough antibodies so that their activity may be assessed in step b).

Ideally, stimulated antibody secreting cells shall be cultured at least 3 days, at least 5 days, at least 10 days, at least 15 days, at least 20 days and even more.

In the preferred embodiment, they are cultured for about 14-15 days.

### IDENTIFICATION OF POOLS CONTAINING AT LEAST A CELL SECRETING AN ANTIBODY HAVING THE DESIRED ACTIVITY AND/OR BINDING PROFILE

In step b), some culture medium of each pool is harvested. This culture medium contains a mixture of the different antibodies that were secreted from the cells present in the pool. It is to be noted that a cell pool is a mixture of expanded cell clones further to the *in vitro* stimulation and amplification of the cells in step a).

These mixtures of antibodies are then assessed in order to identify whether they contain any antibody that present the desired binding and/or biological activity.

Accordingly, the selection of an antibody-secreting cell pool will be carried out based on the determination of the presence in the corresponding cell culture supernatant of an antibody with the desired binding and/or biological activity features.

Preferably, a cell pool will be selected for the presence in the corresponding cell culture supernatant of an antibody binding to one or more antigens and for presenting a particular biological activity.

In particular, where a double selection is performed, i.e., which is based on the binding to one or more antigens and the presence of a biological activity, these tests can be carried out in any order. For example, these can be done as two consecutive selections, i.e., one after the other or both tests carried out in parallel. Preferably, one will first test the cell pools for the presence of an antibody with the binding properties of interest and follow with the selection based on the presence of the desired biological activity.

Many assays may be carried out, notably binding or functional assays can be carried out.

One assay would be to determine whether the culture medium contains any antibody that is able to bind to the target antigen(s). Different techniques are well known from the man skilled in the art, such as: Enzyme Linked Immunosorbent Assay (ELISA), dot-blot, western-blot, immuno-precipitation, flow cytometry or bead-based screening technologies, for example. Multiple binding tests are advantageously carried out. Preferably, said binding test is an ELISA test.

One or more ELISA tests can be carried out to determine the presence in the cell pools supernatants of antibodies with the binding profile of interest. In a particular embodiment, multiple ELISA assays are carried out to select antibody-secreting cells with a binding profile of interest, such as for example, with a differential binding or cross-reacting with different protein variants. The term protein variant has been described above. These multiple ELISA tests can be carried out consecutively, i.e., one after the other, or in parallel.

Examples of particular binding profiles of interest and how the multiple ELISA tests might be carried out for the identification thereof are provided herewith. Multiple ELISA assays can be used, for instance, to identify an antibody cross-reacting with protein variants or proteins with a common conserved motif. In particular, multiple ELISA test can be performed for the selection of monoclonal antibodies that cross-react against a particular protein among species (e.g. human/mouse) or subgroups of the same species (e.g. hemagglutinin or neuraminidase proteins of divergent subtypes of Influenza virus (Clementi N. et al. PLoS One. 2011;6 (12):e28001). Alternatively, multiple ELISA tests can also be carried out to select cell pools containing a B cell secreting an antibody recognizing a phosphorylated epitope but not to the corresponding non-phosphorylated form. For example, by carrying out a first ELISA to select cell pools presenting antibodies specific for the phosphorylated form and subsequently counter-selecting with a second ELISA assay to avoid selecting monoclonal antibodies recognizing the same epitope but non-phosphorylated.

Furthermore, multiple ELISA assays might be carried out to select a cell pool containing in its supernatant a monoclonal antibody recognizing a specific epitope of a particular protein. In one embodiment, ELISA tests are carried out to select monoclonal antibodies against said particular protein and then a second selection against one or multiple peptide sequences is performed to determine whether these antibodies recognize a specific epitope of said protein.

A person skilled in the art will determine the binding tests (i.e., ELISA assays) to be carried out according to the binding profile of interest.

There are many types of functional assays. The man skilled in the art will be able to determine the appropriate one(s) for the target antigen(s). Thus, a particular biological activity will be tested according to the target antigen of choice, this biological activity tests might consist on the determination of a neutralization activity, a cytostatic effect, an Antibody-Dependent Cellular Cytotoxicity (ADCC) activity, a direct cell-killing, phagocytosis, and the like. The functional assays are not always developed, available and/or feasible to identify antibodies against a specific antigen. Below are non-limiting examples of functional assays:
- an assay to assess the biological activity of a target in the presence of no or various amount of antibody;
- an assay to neutralize the biological activity of a target in the presence of no or various amount of antibody; This functional assay is usually used to identify monoclonal antibodies against a viral or bacterial antigen (see for example anti-SARS antibody neutralization assay in WO2004/076677). Additional assays for bacterial antigens could be the serum bactericidal antibody (SBA) and opsonophagocytic assays (OPA) (see for example Romero-Steiner et al. 1997 vol. 4 N°4 pp415-422)
- an assay to determine the activation or inhibition of target cells; the readout may be the activation or inhibition of a signal pathway and/or apoptosis;

In a specific embodiment, the functional activity (i.e., biological activity) of the cell supernatants is determined in step b) with a test measuring the ability of the antibodies in the cell supernatant to neutralize the target protein or organism. An example of neutralization assay against a virus comprises the detection of an antibody which reduces the infectious titre of said virus, i.e., neutralization of virus infectivity. Another example of neutralization test would consist in determining the neutralization of the cytotoxic activity of a cytotoxic toxin.

In a specific embodiment, the desired biological activity is the ability to stimulate ADCC (Antibody-Dependent Cellular Cytotoxicity).

The pools containing at least one cell secreting an antibody with the desired biological activity are thus the pools from which medium containing such an antibody has been harvested.

These pools are then used for the next steps of the method of the invention, which contains steps allowing reduction of the complexity of these pools (which contain 50,000 to 800,000, preferably about 500,000 antibody secreting cells, as seen above) leading to isolation of a specific single cell producing said antibody.

It is to be noted that, most of the time, the pool contains more than one cell secreting an antibody against the specific target. Indeed, there culture condition allowing secretion of antigen often led to clonal expansion of the cells, thereby increasing the number of these cells in the pool.

Nevertheless, these cells are still in minority within the pool in most cases.

### HIGH THROUGHPUT SCREENING OF THE CELL POOLS IDENTIFIED AS CONTAINING ACELL PRODUCING AN ANTIBODY WITH THE DESIRED BIOLOGICAL ACTIVITY AND/OR BINDING PROFILE

In order to identify the cell specifically secreting the antibody of interest, the cells from an identified pool are deposited on an array comprising multiple wells, in such conditions that, statistically, at most three cells are present in each well of said microwell array. Preferably, said array is a microwell array where the size and shape of the wells of said microwell array allow the entry of only a single cell per well.

Prior to applying the cells of a pool to the array, it may be preferable that the wells of the array and the area around them are cleaned with a physiological solution and preferably with culture medium, to replace any previous solution and/or in order to adequately remove impurities, in particular impurities that may have adhered to the surface in the course of forming the coating layer of a binding substance (if such coating layer has been added to the array, as seen below), and improve accuracy of detection.

As indicated above, the cells that are introduced in the wells are part of pools which have been identified to contain at least one cell secreting an antibody of interest, having the desired biological activity and/or binding profile(s) with regards to the target antigen.

### The microwell array

In a preferred embodiment, multiple microwells are disposed in equally spaced rows and columns on a microwell array chip. In the present invention, the terms "chip", "array", "microarray" and "microwell array" will have the same meaning and can be used interchangeably.

Neither the shape nor the size of the microwells is specifically limited. However, for example, the shape of the microwell can be cylindrical. It can also be non cylindrical, such as a polyhedron comprised of multiple faces (for example, a parallelepiped, hexagonal column, or octagonal column), an inverted cone, an inverted pyramid (inverted triangular pyramid, inverted square pyramid, inverted pentagonal pyramid, inverted hexagonal pyramid, or an inverted polygonal pyramid with seven or more angles), or have a shape combining two or more of these shapes. For example, it may be partly cylindrical, with the remainder having the shape of an inverted cone. The bottom of the microwell is usually flat, but curved surfaces (convex or concave) are also possible.

Preferably, said array is a microwell array and the size and shape of the wells of said microwell array allows the entry of only a single cell in each well. Suitable single-cell microwell arrays are described for example in EP1566635, EP1691196 and EP 2184345. For a cylindrically-shaped microwell, the dimensions can be, for example, a diameter of 3 to 100 micrometers. For detecting B lymphocytes, the diameter is desirably 4 to 15 micrometers.

The depth can be from 3 to 100 micrometers, and is desirably 4 to 40 micrometers for detecting B lymphocytes.

The person skilled in the art may determine other shape and size of the microwell, as long as one microwell contains only a single B lymphocyte cell.

To ensure that a B lymphocyte cell will be contained per microwell, one can choose the diameter of the largest circle to fall within a range of 0.5 to 2-fold, desirably 0.8 to 1.9-fold, and preferably, 0.8 to 1.8-fold the diameter of a B lymphocyte. One should also take into consideration whether the diameter of immortalized and/or activated B cells is larger than that of non-immortalized B lymphocytes.

Further, the depth of the microwell preferably falls within a range of 0.5 to 4-fold, desirably 0.8 to 1.9-fold, and preferably, 0.8 to 1.8-fold the diameter of the B lymphocyte cell to be contained in the microwell.

The number of microwells present in a single microwell array chip is not specifically limited. However, when the frequency of a given antigen-specific lymphocyte per 10⁵ cells is from 1 to about 500 at the high end, the number of microwells would range from about 2,000 to 1,000,000 per cm².

### Deposition of the cells in the microwell array

In a specific embodiment, the size of the microwell may allow the entry of more than one cell in each well. In this embodiment, the cells in the pools are diluted in such a way that the concentration of cells in the sample that is applied on the array is such that the amount of medium added in each well of each array contains (statistically) at most three cells.

As a matter of illustration, if a 96 well array is used, and 500 µl is added to each well, the cells are diluted to a concentration of about 6 cells / ml. If 250 µl is added to each well, the cells are diluted to a concentration of about 12 cells / ml. If a 384 well array is used, and 40 µl is added to each well, the cells are diluted to a concentration of at most about 75 cells / ml.

In a preferred embodiment, the dilution is such that each well of each array contains (statistically) at most two cells.

In the preferred embodiment, the dilution is such that each well of each array contains (statistically) at most one cell.

In this embodiment, the distribution of cells in the well of the array (number of cells per well) is generally a normal distribution that is centred on the maximum number (statistically) of cells that is desired in each well. Some wells may contain more or less cells than this number, but the majority of wells contains that number.

In a preferred embodiment, a single-cell microwell array is used, said array is a microwell array where the size and shape of the wells allow the entry of only a single cell per well. The cell pools might be diluted as above-described but preferably, the cells are deposited without dilution. It is particularly preferred that the cells are found in large excess (more cells than microwells) to ensure a good filing rate, i.e., presence of one cell in most of the wells.

### Culture of cells in the array

Typically, a suspension of the cells is deposited on the surface of the array and the cells enter by gravitation into the wells. The cells in the cell pools might have been suspended in culture medium or in a physiological buffer (e.g. Phosphate Buffered Saline (PBS)). Preferably, washing steps are performed prior and/or after the cells deposition in the array with culture medium or with physiological buffer, for example.

Prior to revelation of the wells containing the antibody-secreting cells of interest (i.e., identification of the "spots"), the cells are maintained in the array for a time allowing secretion of the antibody. It should be noted that he culture of the cells in the microarray is not intended to make the cells proliferate, but rather to enable the cells to secrete antibodies.

Preferably, the cells are cultured in a suitable culture medium to ensure both cell viability and antibody secretion in a detectable amount. The culture period can be suitably determined to yield a detectable quantity of produced antibody to bind to the binding substance. Culture may last from a few minutes, to a few hours, or up to 24 hours. Advantageously, the microwell arrays are maintained between 30 minutes and 5 hours, more preferably for 1-3 hours, particularly preferred are incubation times of 2 or 3 hours.

The culture may be performed by covering the array with culture medium. In the case a microwell array with a coating layer of a binding substance is used (as described below), the cells are cultured such as permitting the diffusion, into the coating layer, of the antibodies secreted by said cells. The antibodies that are secreted thus diffuse from the wells into the coating layer around the wells. These antibodies that diffuse and reach the coating layer thus bind to the binding substance comprised in the coating layer.

In a particular embodiment, where a microwell array with a coating layer of a binding substance is used, the following steps will be carried out prior to the cells deposition:
- Day 0: Overnight incubation of the chip with the antigen solution leading to the coating of the chip surface (but not that of the wells because remaining air is a physical obstacle for the antigen solution);
- Day 1: Wash, dispense a saturation solution (e.g., Biolipidure solution) and depressurize the chip to remove the air that is inside each well. Of note, as soon as air is removed, the surface of the wells is now available for the saturation solution and cells will be able to enter the wells. Allow to saturate.
- Day1: Wash the chip and dispense the antibody-secreting cells pool onto the chip.

Preferably this is performed as described in Jin et al. (Nature Protocols, 2011, 6(5) 668-76).

Excessively long culture period results in excessively wide diffusion of the produced antibody, sometimes making it difficult to specify those wells containing cells producing the produced substance. The culture period is desirably suitably determined within a range permitting the ready specification of those wells containing cells producing the produced antibody.

### Isolation of a specific cell producing the antibody with the desired biological activity and/or binding profile from the array

The following step is to identify the wells of the array containing the cell which produces the antibody with the specific biological activity and/or binding profile.

### Use of a coating layer on the array

In one embodiment, the array comprises a coating layer of a binding substance having the ability to bind to at least a portion of a secreted antibody on at least a portion of the principal surface at least around the wells, and wherein the secreted antibodies are enabled to diffuse and bind to said binding substance. Preferably, step d) comprises the step of covering said microwell array with culture medium such that secreted antibody diffuses and binds to the binding substance comprised in the coating layer around the well.

As indicated in EP 2184345, in order to form the coating layer comprising the binding substance, the principal surface of the base plate may be treated with a silane coupling agent, to ensure binding of the binding substance on the principal surface.

Next, a solution containing the binding substance is applied to the surface that has been treated with the silane coupling agent to form the coating layer. The amount of binding substance in the coating layer is determined based on the type of binding substance, and the type of label substance.

The surface treatment to ensure binding of the binding substance to the principal surface is not limited to treatment with a silane coupling agent; any substance promoting binding of a binding substance comprised of protein or the like to the surface of a base plate comprised of an inorganic material (such as a silicon material) or an organic material (such as a polymer material) can be suitably selected for use.

On the surface that is coated with the binding substance, there may be spaces where the binding substance may not be densely present, with portions of uncovered surface remaining depending on the amount of the coating. In such cases, particularly when the surface has been treated with a silane coupling agent as set forth above, there are cases where the antibodies produced by the cells will bind non-specifically to the surface of the base plate. Such nonspecific binding causes a decrease in the precision of detection sensitivity.

Accordingly, in the present invention, it is preferable to apply a blocking agent on the microwell array to coat the portions of the principal surface not covered by the coating layer with the binding substance. An example of a blocking agent is the water soluble polymer Lipidure (registered trademark) having a structural unit in the form of 2-methacryloyloxyethylphosphorylcholine (MPC) with the same structure as the polar base of the phosphatidylcholine constituting the cellular membrane.

### Making an imprint of the array on a solid support

In another embodiment, said step d) comprises the step of making an imprint of the microwell array on a solid support and detecting the presence of antigen-binding antibody on said solid support.

This method has been described in particular in WO2007/035633 and in Love el at (Nat Biotechnol. 2006 Jun;24(6):703-7. Epub 2006 May 14).

In summary, this method may be performed by creating an array of microwells using photolithography and replica molding. Wells are typically 50/100 µm in diameter and depth. The array can be made in PDMS (polydimethylsiloxane), a biocompatible material.

Cells from the pool are deposited on surface of the array, and the excess of medium is removed. The number of cells in each well depends on concentration of cells, volume applied, time, size of wells, and size of the microarray. As indicated above, these parameters may be adjusted such that 0 to 3 cells are present in 10-90% of the wells, depending on the cell pool and the activation procedure. The array is then placed on top of a solid support, pre-treated with a binding substance, which binds to secreted antibodies present in the culture medium.

### Measuring antibody production in the wells

In an alternative embodiment a microwell array without any particular coating is used, preferably said microarray is a single cell microarray for example such as those described in EP1566635 or EP1691196. Those wells wherein an antigen-specific antibody is secreted into the culture medium will be detected by immunochemical measurements. In a particular case, the microwell array is incubated with a solution containing a binding substance to the antibody of interest, for example, said binding substance might be a labelled target antigen.

### Revelation of the wells containing desired cells

Whether the binding substance is present on a coating layer on the array, on a solid support or in the microwells, this binding substance will bind to the secreted antibody.

It may bind specifically to antibodies that bind to the target antigen, or non-specifically to any antibody. It can also bind specifically to only a certain isotype of immunoglobulins (without being specific to antibodies binding the target antigen).

The following step is to use a label substance that will reveal the localization of the wells containing the cell producing the desired antibody. The principle of detection using this label substance may be through competition with the antibody of interest for binding to the binding substance, or through "sandwich" detection similar to ELISA.

In particular, when an array containing a coating layer is used, the label substance is then fed into the coating layer (preferably after removing the culture broth).

Indeed, before feeding the label substance, it is desirable to remove the culture medium. Indeed, for immunoglobulin-producing cells contained in the wells, large amounts of antibody will be secreted into the culture medium. If anti-immunoglobulin antibody is added as a label substance, it may end up binding to the antibody in the culture medium before it binds to the antibody on the chip surface, and may preclude detection of antibody bound to the array surface.

However, there are cases where detection can be conducted without problem by combining cells and binding substances even when the label substance is fed into the coating layer without removing the culture medium.

The label signal is then detected, making it possible to identify the localization of the wells containing the cells that produce or secrete the antibodies of interest.

### Binding substance / label substance

The binding substance may be able to bind to the immunoglobulin of interest.

### Specific binding:

In this case, the binding substance may be the target antigen(s). In this case, the label substance shall be an antibody to the immunoglobulin produced.

### Non-specific binding:

In a first embodiment, the binding substance may be an anti-immunoglobulin antibody, such as an antibody against a constant chain of an immunoglobulin. In this case, the label substance shall be the antigen of interest.

The following table is inspired from Table 1 of EP 2184345 and provides examples of:
(1) substances having the ability to bind to at least a portion of the substance produced by the cells (Binding substance);
(2) substances produced by the cells and which need to be identified (Substance to identify); and
(3) label substances (Needed Label substance) for identifying produced substances.

As will be described below, there are cases where the label substance will bind specifically to the antibody of interest (and presence of said antibody is detected by detection of the label, such as the emitted fluorescence), and cases where it binds specifically to the binding substance (and presence of said produced substance is detected by absence of the label, such as absence of fluorescence).

**Table 1: examples of couples of binding and label substances**

| **Binding substance** | **Substance to identify** | **Needed Label substance** |
|---|---|---|
| Anti-immunoglobulin antibody | Immunoglobulin (antibody) | Antigen |
| Anti-IgG antibody | IgG | Antigen |
| Anti-IgM antibody | IgM | Antigen |
| Anti-IgA antibody | I gA | Antigen |
| Anti-IgE antibody | IgE | Antigen |
| Antigen | IgG | Anti-IgG antibody / Anti-immunoglobulin antibody |
| Antigen | All isotypes of Immunoglobulin | Anti-immunoglobulin antibody |
| Antigen | IgM | Anti-IgM antibody / Anti-immunoglobulin antibody |
| Antigen | IgA | Anti-IgA antibody / Anti-immunoglobulin antibody |
| Antigen | IgE | Anti-IgE antibody / Anti-immunoglobulin antibody |

In a specific embodiment, the binding substance is the target antigen of interest and the label substance is an anti-IgG antibody.

In another embodiment the binding substance is an anti-IgG antibody and the label substance is the target antigen of interest.

### When the binding substance is the antigen

If the binding substance is the antigen, no binding takes place on the coating layer around wells not containing cells producing the antibody of interest.

If an imprint is performed, there will be no binding of antibodies on the imprints of wells not containing cells producing the antibody of interest.

On the other hand, the secreted antibody of interest will bind to the binding substance around the well from which it arises (or to the binding substance on the solid support, at the location of the well from which it arises).

The target antibody produced by a target cell, bound to the binding substance in the coating layer (or on the solid support) is detected by means of the label substance allowing localization of the well containing said target cell.

When the label substance contains a fluorescent moiety, detection of presence or absence of the fluorescence on the array or on the solid support is performed with a fluorescence microscope, fluorescent image scanner, image reader, or the like.

In case of a microwell array with a coating layer, the wells that are labelled (around which binds the label substance) contain cells that secrete the desired antibody.

In case of the imprint method, the label signal spots (also called "spots") can be linked to wells containing cells that secrete the desired antibody.

A particularly preferred approach is the so-called "multiplex" approach wherein monoclonal antibodies reacting against more than one antigen will be detected. In one embodiment, the binding substance will be an anti-immunoglobulin antibody, and the spots will be revealed using various labeled-antigens as labelled substance; this approach was previously described using the ISAAC method by Jin et al. (Nat Med. 2009 Sep;15(9):1088-92; (Nature Protocols, 2011, 6(5) 668-76). In an alternative embodiment, several unlabeled antigens are used as binding substance and the spots are revealed with a labeled-anti-immunoglobulin antibody.

The method of the invention has thus made possible to identify the cell that produces the antibody having the desired biological activity and/or the binding profile against the target antigen.

### CELL RETRIEVAL

The method may also comprise other steps, such as further independently harvesting the cells that are present in the identified wells of step d). Preferably, a labelling method such as a fluorescent CellTracker™ probe is used for cell localisation, thus facilitating the retrieval of the cells contained in the identified wells. The signal from probes is typically monitored using fluorescence microscopy.

Thus, in a preferred embodiment, the method of the invention further comprises the step of independently retrieving the single cell(s) from the identified wells of step d). Preferably, cell retrieval is carried out by micromanipulation techniques which are well known in the art, for example as previously described by Jin et al. (Nature Protocols, 2011, 6(5) 668-76).

If desired, said cells might further be cultured in order to obtain a cell culture. This cell culture shall be obtained through clonal expansion of the single cells present in the identified wells. It is to be noted that where a single-cell microarray has not been used for the cell screening, the cell culture may contain multiple clones, in the case the identified well contained more than one cell. In this case, the steps of cell screening in a microarray may be repeated on said cell culture. Repetition of these steps is optional and, in this case, the cell culture may be processed as if the well only contained a single cell initially.

### VH AND VL SEQUENCES RECOVERY AND CLONING

The method may further comprise the step of isolating (cloning) gene(s) coding for said antibody against said target from said cell culture.

The isolation of VH and VL genes from sorted single cells makes it possible to analyze Ig genes from single B cells and to produce recombinant mAbs. Preferably, the method may further comprise the step of independently isolating the gene coding for said antibody against said target, from the cells present in the identified wells of step d). This would thus be performed without the step of obtaining a cell culture.

Therefore, in a further aspect, the present invention relates to a method for the recovery of the VH and/or VL sequences of a monoclonal antibody comprising the steps of:
a) identifying a cell secreting an antibody against one or more target antigen(s) from a population of cells comprising antibody-secreting cells by using the method described in the first aspect of the invention; and
b) obtaining by RT-PCR the antibody VH and/or VL sequences from said cell.

One of the advantages of the method of the invention is to permit to obtain a high yield of recovery of the antibody of interest from the previously selected cell pool of antibody-secreting cells containing the cell clone of interest (i.e., with the binding/biological activity of interest).

As above-mentioned, the method of the invention comprises the step of depositing the cells from identified cell pools in an array comprising multiple wells. In a preferred embodiment, the cells of each of said identified cell pools are all deposited in at least one array so that all the cells in an array correspond to the same cell pool. In other words, all cells that are present in a given array originate from the same cell pool.

The cells from the pools identified as containing an antibody-secreting cell of interest (i.e., with the desired binding properties/biological activity) will be deposited in one or more arrays, if needed. The microarrays will thus be screened to identify the wells containing cells secreting monoclonal antibodies against the target antigen(s) of interest.

In this embodiment of the method of the invention, each microarray will contain cells originating from an antibody-secreting cells (i.e., B cells) pool selected for its antigen specificity and functional activity (one hit).

Thus, the cells that will be identified, in the microarray, as specific to the antigen (positive spots) have a high probability to originate from a unique B cell clone. Indeed, it is reminded that the purpose of the invention is to identify very rare B cell clones. By stimulating (thus expanding) the cells, one will increase the number of cells that are present in the sample. The expanded cells all originate from an original very rare B cell clone by clonal expansion.

Due to the fact that the positive cells in the microarray have a high probability to originate from the same original B cell, if both VH and VL gene pairs cannot be retrieved from a cell from a single well, it will be possible to clone the VH and the VL chains from cells originating from different wells, originating from the same B-cell clone. Of note that in some cases is possible that one hit contains more than one antibody (VH/VL pair) with the desired binding properties and/or biological activity.

Accordingly, in one embodiment, the antibody VH and VL sequences are both obtained from a cell contained in a single well identified in step d). In an alternative embodiment, the antibody VH and VL sequences are obtained from cells contained in more than one of the wells identified in step d). In this alternative embodiment, the DNA of the VH region is obtained from a cell isolated from a well identified in step d) of the method of the invention and the DNA of the VL region is isolated from a cell isolated from another well identified on the same array.

The fact that, in a single array, many single-cells originating from a single B-cell clone of interest are screened, is a major strength of the method of the invention because it increases the chance to recover the antibody of interest from the selected B-cell Pool.

By contrast, in the methods of the prior art (ISAAC method, as described in Jin et al (Nature Protocols, 2011, 6(5) 668-76)), a single-cell in the array has to be considered as a unique clone thus, there is only a unique opportunity to retrieve both VH and VL gene pairs from the identified hit (see examples and the comparative table ISAAC vs the method of the invention).

Isolation of the genes coding for the antibody of interest is performed, using methods known in the art.
Simonsson et al. (1995, Biotechniques Vol. 18 N°5 pp862-869)
Larrick et al. (1989, biotechniques Vol. 7 pp934-938)
Orlandi et al (1989, Proc. Natl. Acad. Sci. USA vol.86 pp3833-3837).

Furthermore, the isolation of VH and VL genes from sorted single cells was previously described, for example by Babcook et al. (1996, Proc Natl Acad Sci U S A. 23;93(15):7843-8.) or Tiller et al. (2008, J Immunol Methods., 329(1-2):112-24).According to a preferred embodiment, the method used to amplify and isolate from a single B cell the antibody heavy and light chain variable gene sequences is the one of Ozawa et al. (2006, Biotechniques vol. 40 pp469-470, 472, 474).

Indeed, the sequence of all or part of the immunoglobulin-coding gene may be cloned by routine method in the art.

As indicated, the antibody genes may be cloned from a cell culture as obtained after multiplication of the single recovered cells, or directly from the cells present in the identified well (from one single cell or from several cells originating from the same B-cell clone).

Once the genes are isolated, it is possible to produce the antibody in a suitable cell.

It is to be noted that, one would obtain n² possible antibodies, when the identified well contains n cells. Consequently, if the identified well contained more than one cell, (and steps c) and d) are not repeated), it may be needed to produce the all n² antibodies and test each of them for assessing their biological activity. However, this will not be the case when single-cell microarrays have been used.

### METHOD FOR OBTAINING A CELL PRODUCING A MONOCLONAL

### ANTIBODY AGAINST ONE OR MORE TARGET ANTIGEN(S)

The invention also comprises a method for obtaining a cell producing a monoclonal antibody comprising the steps of:
a) isolating mRNA from a cell that is present in the identified wells of step d) of the method as described above, or from a cell culture obtained from one of said cells;
b) performing reverse transcription of said mRNA and amplifying the corresponding cDNA through polymerase chain reaction (RT-PCR);
c) cloning said DNA sequences corresponding to VH and VL regions in a suitable expression vector containing corresponding sequences coding for constant regions CH and CL, thus allowing expression of said VH and VL regions in the context of full length heavy and light chains.

In a particular embodiment the DNA of the VH region has been isolated from a cell isolated from a well identified in step d) of the method of the invention and the DNA of the VL region has been isolated from a cell isolated from another well identified on the same array, in step d) of the method of the invention.

The invention also comprises a method for producing a monoclonal antibody, comprising the step of culturing a cell according to the previous embodiment (or derived from a cell according to the previous embodiment) under such conditions that it expresses said VH and VL regions in the context of an immunoglobulin heavy and light chain, such that in most cases a naturally paired immunoglobulin is produced. A recombinant antibody obtained is to be considered a "naturally paired immunoglobulin" when it is characterised by the binding/functional activity features present in the selected hit from which the retrieved antibody-secreting cells originate from.

The two above methods may also comprise a step of performing the method for identifying a cell secreting the target antibody.

As envisaged herein, a cell derived from a cell according to the previous embodiment is i) a cell that has been obtained through sub-culture of the cell of the previous embodiment, ii) a cell that has been obtained through subcloning (and optional subculturing) of the DNA corresponding to VH and VL that had initially been cloned in the cell of the previous embodiment.

In a preferred embodiment, said cell is a cell from a cell line selected among CHO cells, NS0, HEK293, PerC6, an avian embryonic derived stem cell, such as a chicken embryonic derived stem cell or a duck embryonic derived stem cell and a avian cell line, such as a chicken cell line or a duck cell line.

Generation of genetically modified cells to express recombinant proteins is well-known by the man skilled in the art. Methods which are well known to and practiced by those skilled in the art can be used to construct expression vectors containing sequences encoding the proteins and polypeptides of interest, as well as the appropriate transcriptional and translational control elements. These methods include in vitro recombinant DNA techniques, synthetic techniques, and *in vivo* genetic recombination. Such techniques are described for example in Sambrook et al. (1989, Molecular Cloning, A Laboratory Manual, Cold Spring Harbor Press, Plainview, N.Y.) and in Ausubel et al. (1989, Current Protocols in Molecular Biology, John Wiley & Sons, New York, N.Y.).

The examples below explain the invention in more detail. The following preparations and examples are given to enable those skilled in the art to more clearly understand and to practice the present invention. The present invention, however, is not limited in scope by the exemplified embodiments, which are intended as illustrations of single aspects of the invention only, and methods which are functionally equivalent are within the scope of the invention. Indeed, various modifications of the invention in addition to those described herein will become apparent to those skilled in the art from the foregoing description and accompanying drawings. Such modifications are intended to fall within the scope of the appended claims.

In particular, The examples are intended to illustrate the advantages of the method with regards to a method of the prior art. This method was used to identify antibodies against a bacterial and a viral pathogens. The names of the target are not disclosed for confidentiality reasons. Furthermore, since the object of the invention is the method as described in the examples, which is usable on various targets, the names of the targets are not relevant to illustrate the method.

### EXAMPLES

### Example 1: Materials and Methods

### Bacterial toxin 1 and toxin 2 ELISA

Human IgG specifically binding to toxin 1 or toxin 2 of a gram positive bacteria were detected by ELISA. A said gram positive bacteria is a pathogenic bacteria, the name of which is not disclosed for confidentiality reasons. To this end, 96-wells ELISA plates (GREINER) were coated overnight at +4°C with 0.5 µg/mL (100µL/well) purified toxin 1 or toxin 2 in carbonate buffer pH 9.6. Plates were then washed twice with 250 µL/well of PBS, 0.05% Tween-20 (SIGMA ALDRICH) and saturated 2 hours at room temperature with 200 µL/well of PBS, 0.05% Tween-20, 1% BSA (MP-Bio). Plates were then washed twice with 250 µL/well of PBS, 0.05% Tween-20 before addition of samples to be tested.

For detection of IgG in supernatants harvested from activated B cell pools, 100 µL of supernatants diluted ½ in PBS, 0.05% Tween-20, 0.1% BSA were incubated for 1 hour at room temperature on toxin 1 or toxin 2 coated plates. Plates were then washed 3 times with 250 µL/well of PBS, 0.05% Tween-20, and 100 µL/well of a HRP-conjugated rabbit anti-human IgG antibody (DAKO) were added. After 1 hour at room temperature, plates were washed 3 times with 250 µL/well of PBS, 0.05% Tween-20, and 100 µL/well of HRP substrate (TMB, KPL) were added. After 30 minutes incubation the enzymatic reaction was stopped by addition of 100 µL/well of 1 N ortho-phosphoric acid (Merck). The presence of IgG binding to toxin 1 or toxin 2 in a well was then measured by reading the optical density (OD) at 450 nm on a VersaMax ELISA plate reader (Molecular Devices).

For titration of recombinant monoclonal antibodies (hereinafter "mAbs"), 100 µL of serial dilution in PBS, 0.05% Tween-20, 0.1% BSA of unpurified supernatants or purified human IgG1 from CHO cells transfected with plasmids coding the H and L chains of the mAbs to be tested, were added into wells of ELISA plates coated with toxin 1 or toxin 2. The ELISA was performed essentially as described above. Samples were tested in duplicate and the results were expressed as the mean OD.

### Bacterial toxin 1 and toxin 2 cytotoxicity assay.

The capacity of antibodies to neutralize *in vitro* the functional activity of bacterial toxin 1 or toxin 2 was determined by using a cell-based cytotoxicity assay with human IMR-90 cells. The day before antibody testing, IMR-90 cells (ATCC CCL-186) were seeded at 10,000 cells/well in flat bottom 96 half-well culture plates (GREINER) under 50µL/well and incubated at 37°C, 5% CO₂. Samples to be tested for the presence of neutralizing antibodies (unpurified supernatants of activated B cell pools, unpurified or purified antibodies from CHO cells transfected with plasmids coding antibody heavy (H) and light (L) chains) were pre-incubated for 1 hour at 37°C with toxin 1 or toxin 2 used at concentrations previously determined to be sub-optimal in the cytotoxicity assay. For screening of functional hits, supernatants of B cell pools were tested at ½ final dilution. To measure the neutralizing potency of antibodies, serial dilutions of recombinant unpurified or purified mAbs from CHO cells were tested. After 1 hour pre-incubation, the antibody-toxin mixtures were transferred into wells of plates containing the IMR-90 cells and plates were incubated at 37°C, 5% CO₂. After 24 hours incubation, the cytotoxic activity of the toxins was evaluated by examination of IMR-90 cells under microscopy (round cells being died, adherent cells being alive) and counting the % of died cells. Samples were tested in duplicate and results were expressed as mean % of cell death.

### Detection of antibodies with multi antigen binding properties by ELISA

Human IgG binding to viral protein X variant A, viral protein X variant B, and to 4 peptides spanning two partially overlapping regions of both viral protein X variants (i.e., Peptide 1, variant A; Peptide 2, variant A; Peptide 1, variant B; Peptide 2, variant B) were detected by ELISA. Virus X represents a pathogenic virus, the name of which is not disclosed for confidentiality reasons.

To this end, 96-wells ELISA plates (Corning) were coated under 100 µL/well overnight at +4°C with 0.5 µg/mL purified viral protein X variant A or viral protein X variant B, or with 5 µg/mL Peptide 1, variant A; Peptide 2, variant A; Peptide 1, variant B or Peptide 2, variant B in carbonate buffer pH 9.6. Plates were then washed 4 times with 250 µL/well of PBS, 0.05% Tween-20 (SIGMA ALDRICH) and saturated 2 hours at room temperature with 200 µL/well of PBS, 0.05% Tween-20, 3% BSA (SIGMA). Plates were then washed 4 times with 250 µL/well of PBS, 0.05% Tween-20 before addition of samples to be tested.

For detection of IgG in supernatants harvested from activated B cell pools, 100 µL of supernatants diluted ½ in PBS, 0.05% Tween-20, 1% BSA were incubated for 1 hour at room temperature on proteins or peptides coated plates. Plates were then washed 4 times with 250 µL/well of PBS, 0.05% Tween-20, and 100 µL/well of biotinylated goat anti-human IgG (Southern Biotech) were added. After 1 hour at room temperature, plates were washed 4 times with 250 µL/well of PBS, 0.05% Tween-20, and 100 µL/well of streptavidin-HRP (KPL) was added. After 1 hour incubation, 100 µL/well of HRP substrate (TMB, KPL) were added. After 10 minutes incubation the enzymatic reaction was stopped by addition of 100 µL/well of TMB Stop solution (KPL). The presence of IgG binding to Proteins and/or Peptides in a well was then measured by reading the optical density (OD) at 450 nm on a VersaMax ELISA plate reader (Molecular Devices).

For titration of recombinant mAbs, 100 µL of serial dilution in PBS, 0.05% Tween-20, 1% BSA of purified human IgG1 from CHO cells transfected with plasmids coding the H and L chains of the mAbs to be tested, were added into wells of ELISA plates coated with Proteins or Peptides. The ELISA was performed essentially as described above. Samples were tested in duplicate and the results were expressed as the mean optical density (OD).

### Example 2: Identification and generation of human monoclonal antibodies neutralizing bacterial toxin 2 using the B cell screening method of the invention

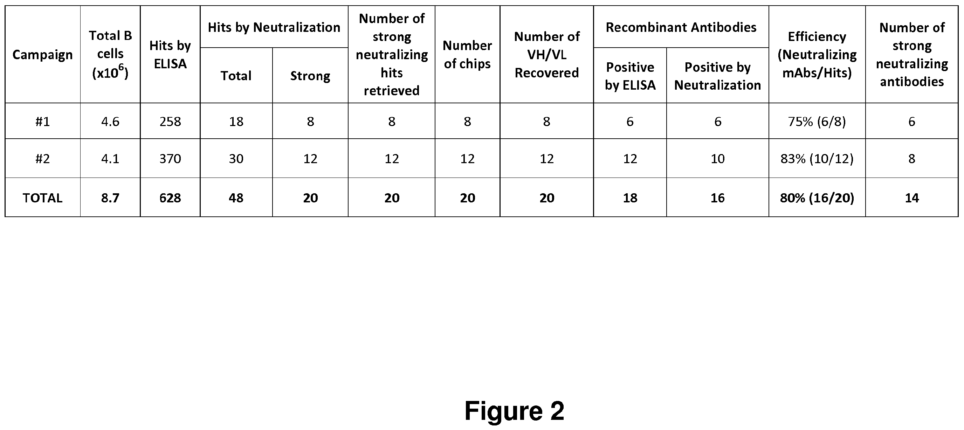

Peripheral blood mononuclear cells (PBMCs) were obtained by Ficoll® centrifugation (Lymphocytes Separation Medium, Eurobio) from a blood donor, in a particular donor, that was previously screened positive for the presence of serum IgG binding to toxin 2 of gram positive bacteria and for neutralizing toxin 2 cytotoxic activity. 65 million of PBMCs containing about 7% of B cells, that is to say about 4.6 millions of B cells, were used in the first screening campaign (campaign #1 in **Figure 2**). The B cells in this population were further enriched in IgG+ B cells by depleting IgM/IgD-expressing B cells with mouse anti-human IgM plus anti-human IgD antibodies cocktail (Southern Biotech) followed by magnetic beads coated with anti-mouse IgG antibodies (Dynabeads Pan Mouse IgG, Invitrogen,). The obtained PBMC population depleted in IgM/IgD+ B cells contained approximately 0.4 million IgG+ B cells.

The IgG+ enriched B cells were infected with Epstein-Barr virus (EBV, prepared from the B95-8 cell line (ECACC 85011419) by Vivalis) for 45 minutes prior to CD40L stimulation. Cells were seeded at a cell density of 200 IgG+ B cells/well and cultured during 14 days in 96-well flat bottom culture plates. During that period, B cells proliferated leading to 100,000-500,000 cells/well and secreted IgG antibodies to an expression level in the culture supernatant of 5 to 20µg/mL.

At day 14, supernatants were harvested from each culture well and tested by ELISA for detection of IgG binding to bacterial toxin 2. In total, 258 supernatants were found positive by ELISA and were further tested for their capacity to neutralize toxin 2 cytotoxicity in a functional assay (**Figure 2**). Among 18 supernatants found positive in the functional assay, 8 displayed strong neutralization activity. These results show that B cell pools producing antibodies with the desired specificity and functionality can be identified after their *in vitro* activation and amplification with EBV/CD40L combination.

Samples of cells from the 8 strongly positive culture wells were then loaded on microarrays for single cell screening against bacterial toxin 2 using ISAAC technology as described in Jin et al (Nature Protocols, 2011, 6(5) 668-76), so that to each single positive culture well corresponded to one single microarray. These microarrays are characterized by having wells of a size and shape so that they contain a maximum of one single cell per well and in this particular case the surface surrounding the microwells was coated with bacterial toxin 2. After 3h incubation, the presence of IgG specifically binding to toxin 2 was revealed by staining with a Cy3-conjugated anti-human IgG antibody (SIGMA ALDRICH) and fluorescence microscopy examination (NIKON Eclipse 90i equipped with appropriate fluorescent filters). Single B cells secreting IgG specific to toxin 2 were therefore identified by the presence of a fluorescent spot around the wells of the microarrays.

It is to be noted that, due to the cell amplification linked to the *in vitro* B cell stimulation carried out during 14 days, each positive culture well in 96 well flat bottom culture plates should contain approximately between 0.5% and 2% of cells expressing the antibody of interest at the end of 14 days long culture. The microarray comprises 62,500 wells. Assuming that at least 50% of the wells are loaded with a single cell (i.e average filing rate), approximately 30,000 cells can thus be assessed for their ability to secrete the antibody of interest. It is thus expected that approximately between 150 and 600 cells expressing the antibody of interest are present on the microarray. This number should guarantee that it is possible to further identify the sequence of said antibody (taking into account any loss while recovering the single cells and/or cloning the sequence of the antibody).

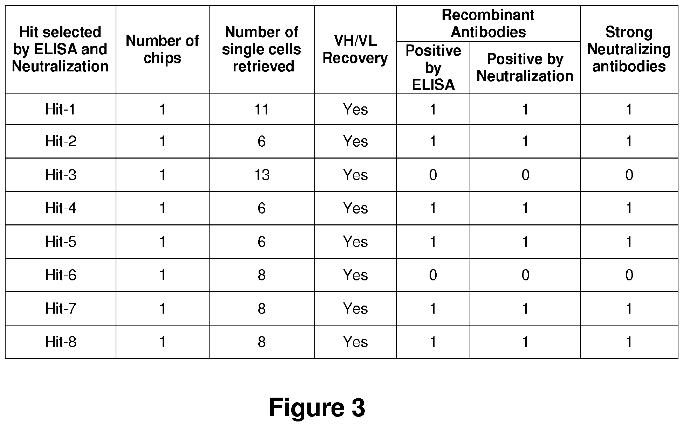

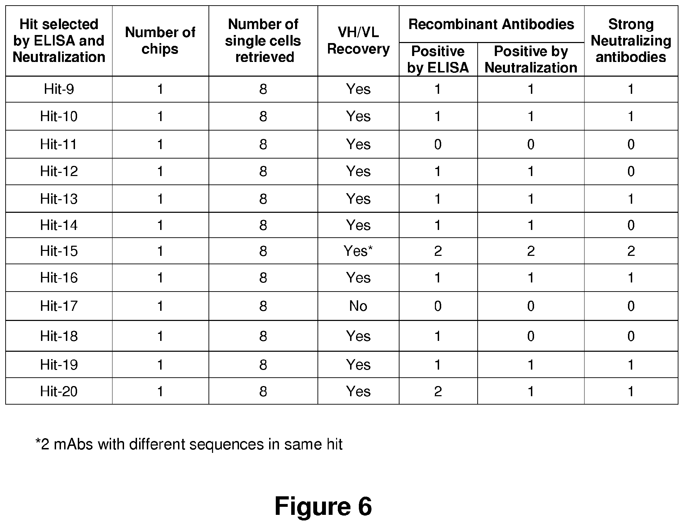

Spots revealing the antigen-antibody interaction were detected on the 8 microarrays, that is, for each of the 8 B cell pools previously selected. Between 6 to 13 single cells producing anti-toxin 2 IgGs were retrieved by micromanipulation as described by Jin et al.(Nature Protocols, 2011, 6(5) 668-676) from each of the 8 microarrays (**Figure 3**). The sequence of the variable region of the heavy (VH) and light (VL) chains of the antibodies produced by the selected single cells were obtained by RT-PCR also as described by Jin et al.(Nature Protocols, 2011, 6(5) 668-676). VH and VL cDNA sequences were individually cloned in an expression vector for the production of full-length IgG1 H and L chains and co-transfected into CHO cells. Supernatants of said CHO cells transiently co-transfected with H and L expression plasmids were collected and tested for their ability to bind to toxin 2 and to neutralize its cytotoxicity. As shown in **Figure 3**, 6 out of 8 supernatants were confirmed to contain recombinant human IgG binding to toxin 2 in ELISA. The same supernatants were also capable to neutralize the toxin 2 toxicity by functional assay.

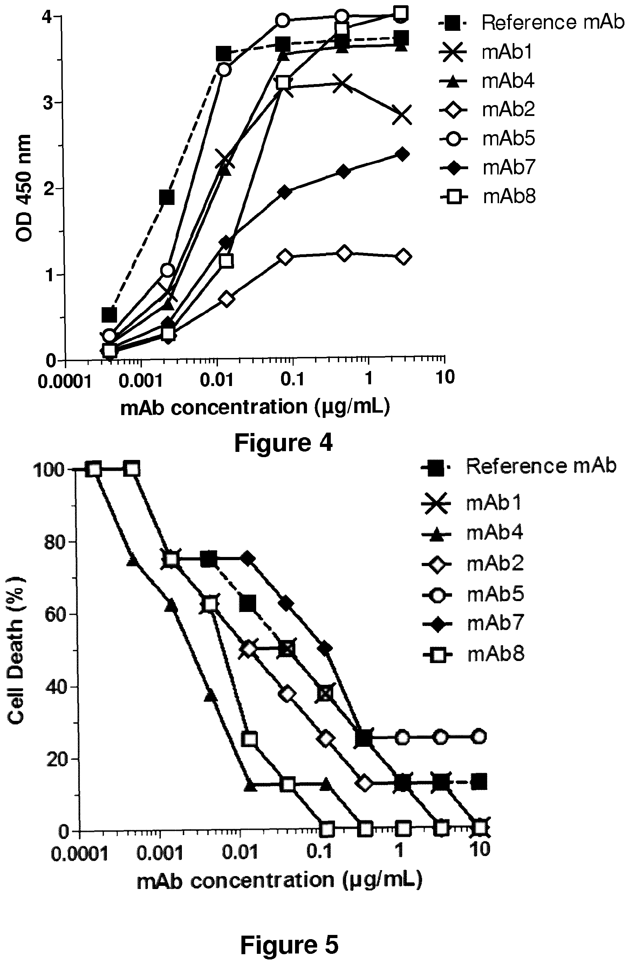

The 6 neutralizing antibodies were further produced by transfection in CHO cells and purified on protein A column (HiTrap mAbSelect Sure, GE Healthcare). Titration by ELISA of the 6 purified recombinant monoclonal antibodies showed that 4 of them were strongly binding to toxin 2 and 2 more weakly (**Figure 4**). Likewise, the cytotoxic activity of the 6 purified antibodies was determined by cytotoxicity assay as described in Example 1. The titration results showed that all 6 mAbs strongly neutralized the toxin 2 cytotoxicity, and 3 of them were superior to a strong toxin 2 neutralizing reference antibody currently in late stage clinical development (**Figure 5**). Accordingly, in this example, the method of the invention enabled 75% efficiency between the selection of B cell pools with the biological activity of interest and the number of functional antibodies isolated, namely 6 out of 8 selected B cell pools (hits).

In a second campaign performed with 4.1 million B cells from the same donor (campaign #2, **Figure 2**), 10 neutralizing recombinant antibodies were generated starting from 12 B cell pools displaying strong neutralizing activity against toxin 2 (**Figure 6**). Among the 10 neutralizing antibodies, 8 strongly neutralized toxin 2 cytotoxicity (**Figure 2**, last row). Of note, 2 neutralizing antibodies with different VH and VL sequences were cloned from a same B-cell pool (Hit-15) (**Figure 6**).

Altogether (campaigns #1 and #2), the method of the invention enabled 80% efficiency between the selection of B cell pools with the biological activity of interest and the number of functional antibodies isolated, namely 16 out of 20 selected pools (hits).

### Example 3: Identification and generation of human monoclonal antibodies neutralizing bacterial toxin 2 using the ISAAC technology

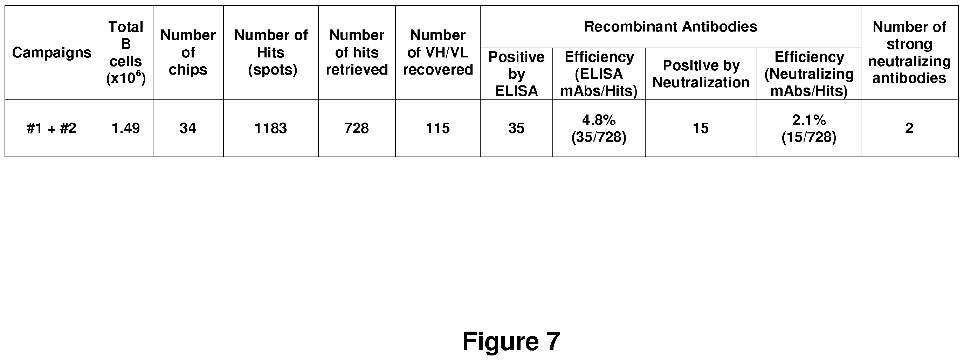

The ISAAC technology for single cell screening was used essentially as described in Jin et al (Nature Protocols, 2011, 6(5) 668-676) to generate human monoclonal antibodies (mAbs) against toxin 2. Accordingly, 21.4 million PBMC purified from the same donor as in Example 2 (which contained 7% of B cells, that is to say 1.49 million of B cells) were activated in bulk in 2 campaigns with a cocktail of R-848 (Enzo Life Sciences) and IL-2 (Peprotech). After 5 days of culture, activated B cells were collected and loaded on 34 microarrays (**Figure 7**) coated with toxin 2 as described in Jin et al (Nature Protocols, 2011, 6(5) 668-676). After 3h incubation, the presence of IgG specifically binding to toxin 2 was revealed by staining with a Cy3-conjugated anti-human IgG antibody and fluorescence microscopy examination. Single B cells secreting IgG specific to toxin 2 were therefore identified by the presence of a fluorescent spot around the wells of microarrays.

As shown in **Figure 7**, 1183 anti-toxin 2 spots were detected on the 34 microarrays analyzed. With the ISAAC approach each single cell corresponds to 1 hit, as B cells have not been previously pre-sampled in 96-well plate and in vitro amplified. Among the 1183 hits detected, 728 single cells were retrieved and processed for VH and VL sequences isolation and cloning. 115 VH/VL pair sequences were recovered (**Figure 7**), individually cloned in expression vectors for the production of full-length IgG1 H and L chains and CHO cells were transiently co-transfected with pairs of H and L expression plasmids. CHO supernatants were further collected and tested for their ability to bind to toxin 2 and to neutralize toxin 2 cytotoxicity. As shown in **Figure 7**, 35 out of 115 supernatants were found to contain recombinant human IgG binding to toxin 2 in ELISA. Moreover, 15 of the same 115 supernatants were also capable to neutralize the toxicity of toxin 2.

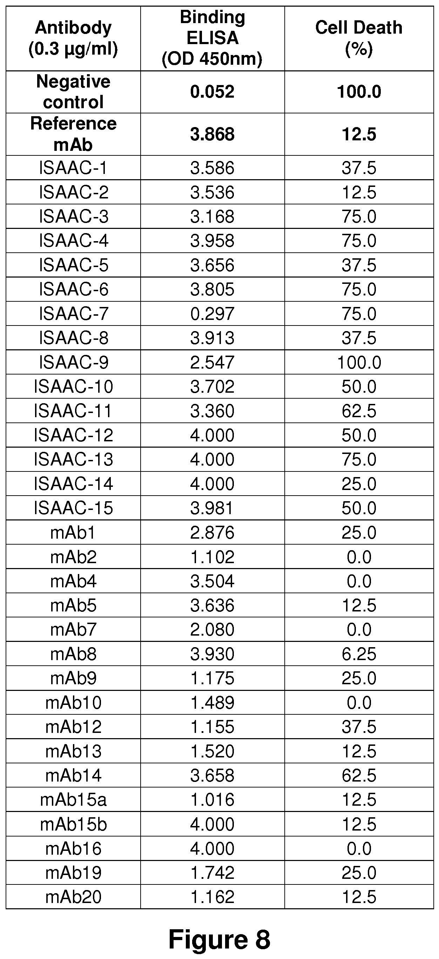

These results show that 35 antibodies binding to toxin 2 could be generated from 728 single B cells (hits) selected only by binding on microarrays (4.8% efficiency). However, 15 of the 35 anti-toxin 2 mAbs demonstrated a neutralizing activity and only 2 mAbs (ISAAC-2 and ISAAC-14) displayed strong neutralizing activity comparable to that of a strong toxin 2 neutralizing reference antibody currently in late stage clinical development (**Figure 8**). In contrast, when tested in the same conditions, all but 2 functional mAbs (mAb12 and mAb14) obtained by the method of the invention displayed neutralizing activity comparable or superior to the reference mAb (**Figure 8**).

Accordingly, in this particular example, the ISAAC methodology enabled 2.1% efficiency in the selection through single cell screening of antibodies with toxin 2 neutralizing activity, namely a total of 15 out of the 728 single cells (hits) identified as producing antibodies specific against toxin 2 in binding assay.

Thus, as illustrated in **Figure 9** summarizing data obtained with the screening method of the invention versus ISAAC campaigns with PBMC from the same donor, the probability to identify and to clone monoclonal antibodies with strong functional activity is highly enhanced with the screening method of the invention, which comprises a first amplification step (whereby sampled B cells proliferate and produce antibodies) and the early selection of B cell pools producing antibodies with the desired specificity and functionality. Moreover, the probability to obtain antibodies with strong functional activity is highly enhanced by the early selection of B-cell pools producing antibodies with the strongest biological activity as obtained with the screening method of the invention.

### Example 4: Identification and generation of human monoclonal antibodies neutralizing bacterial toxin 1 using B cell screening method of the invention

A B cell screening campaign using the method of the invention, as described in Example 2 above, was carried out enabling the identification of 2 B cell pools (hits) against toxin 1 of the same gram positive bacteria of Example 2, namely hit N°21 and hit N°22, based on the presence in the activated B cells supernatants of human IgGs binding to toxin 1, as determined by ELISA, and neutralizing activity against toxin 1 cytotoxicity.

Each of these B cell pools was deposited on a single-cell microarray were the surface surrounding the wells was coated with toxin 1, and single B cells secreting specific human IgG against toxin 1 were detected and retrieved, essentially as described in Example 2.

Hit N° 21: 8 positive single cells were retrieved from the microarray of the hit N°21. Out of the 8 cells, VH and VL gene pairs were successfully amplified by RT-PCR from 5 single cells (**Figure 10A**). The 5 VH/VL gene pairs were cloned in expression vectors and transfected in CHO cells. All supernatants of the transfected CHO cells were positive by ELISA against toxin 1. Unique VH and VL consensus sequences were found from the 5 single cells that led to positive ELISA signals and the corresponding plasmids from one of these single cells, namely the gamma chain N°3G and kappa chain N°3k, were used to produce a recombinant antibody at the mg scale in CHO cells. The purified recombinant candidate was confirmed to bind toxin 1 by ELISA and to neutralize toxin 1 by the cytotoxicity neutralization functional assay.

Hit N° 22: for this hit, only 3 cells could be retrieved from the microarray. In this case, the VH gene was amplified from the single cell N°2, while the VL gene was successfully amplified from cells N°1 and N°3 (**Figure 10B**). Thus, in contrast to the hit N°21, no pair of VH and VL genes could be recovered from any of the 3 single cells. Therefore, to recover the VH and VL pair from that hit, after cloning in expression vectors, the gamma chain containing the VH sequence from cell #2 was tested in combination with both the light chain containing the VL sequence from cell #1 and cell #3. As shown in **Figure 10B****,** the supernatant of CHO cells transfected with the gamma chain isolated from the single cell N°2 (gamma chain N°2G) and the light chain isolated from the single cell N°3 (Light chain N°3L) was positive by ELISA against toxin 1. The corresponding plasmids were used to produce a recombinant antibody at the mg scale in CHO cells. The purified recombinant mAb confirmed to bind toxin 1 by ELISA and to neutralize toxin 1 toxicity.

Accordingly, the screening method of the invention enables obtaining a VH/VL pair by the combination of VH and VL sequences originating from more than one single cell in the microarray, as all the cells in the microarray correspond to one previously selected pool of B cells (one hit).

By In the ISAAC method described by Jin et al (Nature Protocols, 2011, 6(5) 668-676), each spot in the microarray corresponds to a single cell producing a unique antibody, therefore the successful isolation of the antibody can only be achieved when both VH and VL sequences are recovered from that single cell.

However, with the cell screening method of the invention, each microarray will contain cells originating from a B cell pool selected for its antigen specificity and functional activity (one hit). Thus, the single cells in the microarray identified as specific to the antigen (positive spots) will have a high probability to belong to a unique B cell clone. Accordingly, even by incomplete VH/VL amplification (VH or VL from a single cell), as shown above for the hit N°22, it will be possible to successfully recover the candidate antibody sequence. Thus, the cell screening method of the invention increases the efficiency of recovery of both VH and VL sequences from the targeted B cell clones.

### Example 5 : Identification and generation of human monoclonal antibodies presenting multi antigen binding properties with the B cell screening method of the invention

To generate human monoclonal antibodies showing distinct multi-antigen binding profiles, 60 million PBMCs from two donors (I and II), which had been previously screened positive for the presence of serum IgG binding to the viral protein X variants A and B, were enriched in IgG+ B cells by depleting IgM/IgD-expressing B cells with mouse anti-human IgM plus anti-human IgD antibodies cocktail followed by magnetic beads coated with anti-mouse IgG antibodies, as previously disclosed in Example 2.

The IgG+ enriched B cells were then stimulated by EBV/CD40L, seeded at a cell density of 200 IgG+ B cells/well and cultured during 14 days in 96-well culture plates. At day 14, supernatants were harvested from each culture well and tested by ELISA for detection of IgG binding to the antigens of interest. Supernatants from B cell pools were screened by ELISA against Protein X variants A and B, and against 4 peptides spanning two partially overlapping regions of both Protein X variants (i.e., Peptide 1, variant A; Peptide 2, variant A; Peptide 1, variant B; Peptide 2, variant B). Supernatants from 38 B cell pools (donor I) and 17 B cell pools (donor II) were found positive for the presence of antibodies recognizing the Protein X variants. All 55 candidates were further tested by peptide ELISA. Among these candidates, 2 hits were found with the targeted binding profiles which were: i) Protein X variant A, and peptides 1 and 2 variant A; and ii) Protein X variants A and B, and peptide 2 for both variants. Results are shown in **Figure 11****.**

Samples of cells from these 2 positive culture wells were then loaded on microarrays coated with a mixture of Protein X A and B variants, as described in Jin et al (Nature Protocols, 2011, 6(5) 668-676), so that to each single positive B cell pool was loaded into one microarray for single cell screening. After 3h incubation, the presence of IgG specifically binding to Protein X A and B variants was revealed by staining with a Cy3-conjugated anti-human IgG antibody and fluorescence microscopy examination. Single B cells secreting IgG specific to Protein X A and B variants were therefore identified by the presence of a fluorescent spot around the wells of microarrays.

VH/VL gene pairs were obtained by RT-PCR from the isolated single cells from both hits and corresponding sequences cloned into expression vectors. CHO cells were then transfected for monoclonal antibody production and characterization. 6 days after transfection, CHO supernatants were screened by ELISA. As indicated on **Figure 11****,** the 2 recombinant antibodies showed binding profiles that are indistinguishable to that of the corresponding antibodies produced by initial B cell pools of interest.

The hit N°1 recombinant antibody, was produced in CHO cells, purified by protein A chromatography and then dialyzed against PBS, pH7.4. Its antigenic specificity for the 6 antigens was then assessed by ELISA. As shown in **Figure 11****,** the purified IgG1 showed binding to both variants of the viral protein X, with a strong binding against the peptide 2, but not the peptide 1, in agreement with the profile of the selected hit (**Figure 11**).

The use of the cell screening method of the invention therefore allows the early selection of hits with a very specific target profile and the subsequent isolation of the corresponding VH and VL sequences.

## Claims

1. A method for identifying, in a population of cells comprising antibody-secreting cells, a cell secreting an antibody against one or more target antigen(s), said method comprising the steps of:
a) *in vitro* stimulation and amplification of the cells of said population and cell sampling in order to obtain multiple cell pools which contain expanded cell clones;
*b)* screening of said cell pools supernatants to identify and select those cell pools containing cells secreting antibodies binding to said target antigen(s) and/or presenting a biological activity;
c) depositing the cells of said identified cell pools in an array comprising multiple wells in such conditions that at most three cells are present in each well of said array;
d) culturing the cells in the wells of said array and screening thereof to identify those wells containing a cell secreting an antibody against said target antigen(s).

2. The method of claim 1, wherein in step c) all the cells that are present in a given array originate from the same cell pool.

3. The method of any of claims 1 or 2, wherein said cell population has been infected with a lymphotropic virus before or during step a).

4. The method of any of claims 1 to 3, wherein after cell sampling, said cell pools are cultured in a culture medium under conditions in which the cells secrete antibodies into said culture medium.

5. The method of any of claims 1 to 4, wherein in step b) the cell pool supernatants are selected for the presence of an antibody binding to said one or more target antigens and for presenting a biological activity.

6. The method of any of claims 1 to 4, wherein in step b) the cell pool supernatants are selected for the presence of an antibody with a binding profile of interest by carrying out multiple binding assays.

7. The method of any of claims 1 to 6, wherein the size and shape of the wells of said array in step c) allow the entry of only a single cell per well.

8. The method of any of claims 1 to 7, wherein said array in step c) comprises a coating layer of a binding substance on at least the surface around the wells, wherein said binding substance has the ability to bind to at least a portion of a secreted antibody and wherein the secreted antibodies are enabled to diffuse and bind to said binding substance.

9. The method of any of claims 1 to 8, wherein step d) comprises the step of making an imprint of the array on a solid support and detecting the presence of antigen-binding antibody on said solid support.

10. The method of any of claims 1 to 9, wherein said population of cells comprising antibody-secreting cells is from a human being.

11. The method of claim 8, where said binding substance is one or more of said target antigen(s) or an anti-immunoglobulin antibody.

12. The method of any of claims 1 to 11, further comprising the step of independently retrieving the single cell(s) from the identified wells of step d).

13. A method for the recovery of the VH and/or VL regions DNA of an antigen-specific monoclonal antibody comprising the steps of:
a) performing the method of any of claims 1 to 12 in order to identify a cell secreting an antibody against a target antigen; and
b) obtaining by RT-PCR the DNA of the VH and/or VL regions wherein
i. the DNA of the VH and VL regions has been obtained from a cell isolated from a single well identified in step d) of the method of any of claims 1 to 12, or
ii. the DNA of the VH region has been obtained from a cell isolated from a well identified in step d) of the method of any of claims 2 to 12 and the DNA of the VL region has been isolated from a cell isolated from another well identified on the same array, in step d) of the method of any of claims 2 to 12.

14. A method for obtaining a cell producing an antigen-specific monoclonal antibody comprising the steps of:
a) isolating mRNA from a cell that is present in the identified wells of step d) of the method of claims 1 to 12, or from a cell culture obtained from such cell;
b) performing reverse transcription of said mRNA and amplifying the corresponding cDNA through RT-PCR;
c) cloning said DNA sequences corresponding to VH and VL regions in a suitable expression vector.

15. A method for producing a monoclonal antibody comprising the steps of:
a) performing the method of any of claims 1 to 12 to identify a cell secreting an antibody against a target antigen;
b) isolating VH and VL genes encoding the antibody from the cell of step a) or its cell culture and cloning these genes; and
c) expressing the genes of step b) in a suitable cell to obtain the monoclonal antibody.

## Patentansprüche

1. Verfahren zum Identifizieren in einer Population von Zellen, die antikörpersezernierende Zellen umfassen, einer Zelle, die einen Antikörper gegen ein oder mehr Zielantigen(e) sezerniert, wobei das Verfahren die folgenden Schritte umfasst:
a) In vitro-Stimulation und Amplifikation der Zellen der Population und Zellprobenentnahme, um mehrere Zellpools zu erhalten, die expandierte Zellklone enthalten;
b) Screenen der Zellpool-Überstände, um solche Zellpools zu identifizieren und zu selektieren, die Antikörper sezernieren, welche an das bzw. die ZielAntigen(e) binden und/oder eine funktionelle Aktivität aufzeigen;
c) Ablagern der Zellen der identifizierten Zellpools in einem Array, der mehrere Vertiefungen umfasst, unter solchen Bedingungen, dass höchstens drei Zellen in jeder Vertiefung des Arrays vorhanden sind;
d) Züchten der Zellen in den Vertiefungen des Arrays und Screenen derselben, um diejenigen Vertiefungen zu identifizieren, die eine Zelle, die einen Antikörper gegen das bzw. die Antigen(e) sezerniert, enthalten.

2. Verfahren nach Anspruch 1, wobei in Schritt c) sämtliche Zellen, die in einem bestimmten Array vorhanden sind, aus dem gleichen Zellpool stammen.

3. Verfahren nach einem der Ansprüche 1 oder 2, wobei die Zellpopulation vor oder während Schritt a) mit einem lymphotropen Virus infiziert worden ist.

4. Verfahren nach einem der Ansprüche 1 bis 3, wobei die Zellpools nach der Zellprobenentnahme in einem Kulturmedium unter Bedingungen gezüchtet werden, bei denen die Zellen Antikörper in das Kulturmedium sezernieren.

5. Verfahren nach einem der Ansprüche 1 bis 4, wobei in Schritt b) die Zellpool-Überstände auf die Anwesenheit eines Antikörpers, der an ein oder mehr Antigene bindet, und auf das Aufzeigen einer biologischen Aktivität selektiert werden.

6. Verfahren nach einem der Ansprüche 1 bis 4, wobei in Schritt b) die Zellpool-Überstände auf das Vorhandensein eines Antikörpers mit einem Bindungsprofil von Interesse selektiert werden, indem mehrere Bindungstests durchgeführt werden.

7. Verfahren nach einem der Ansprüche 1 bis 6, wobei die Größe und Form der Vertiefungen des Arrays in Schritt c) den Eintritt von nur einer einzigen Zelle pro Vertiefung ermöglichen.

8. Verfahren nach einem der Ansprüche 1 bis 7, wobei der Array in Schritt c) eine Überzugsschicht aus einer bindenden Substanz auf mindestens der Oberfläche um die Vertiefungen herum umfasst, wobei die bindende Substanz zumindest an einen Teil eines sezernierten Antikörpers binden kann und wobei die sezernierten Antikörper diffundieren und an die bindende Substanz binden können.

9. Verfahren nach einem der Ansprüche 1 bis 8, wobei Schritt d) den Schritt umfasst, bei dem ein Abdruck des Arrays auf einem festen Träger vorgenommen wird und das Vorhandensein von antigenbindendem Antikörper auf dem festen Träger erfasst wird.

10. Verfahren nach einem der Ansprüche 1 bis 9, wobei die Population von Zellen, die die antikörpersezernierenden Zellen umfassen, aus einem Menschen stammt.

11. Verfahren nach Anspruch 8, wobei die bindende Substanz ein oder mehr von dem bzw. den Zielantigen(en) oder eines Anti-Immunglobulin-Antikörpers ist.

12. Verfahren nach einem der Ansprüche 1 bis 11, zudem umfassend den Schritt, bei dem die einzelne(n) Zelle(n) aus den identifizierten Vertiefungen von Schritt d) erfasst wird bzw. werden.

13. Verfahren zur Gewinnung der DNA der VH- und/oder VL-Regionen eines antigenspezifischen monoklonalen Antikörpers, umfassend die folgenden Schritte:
a) Durchführen des Verfahrens nach einem der Ansprüche 1 bis 12 zum Identifizieren einer Zelle, die einen Antikörper gegen ein Zielantigen sezerniert; und
b) Erhalten der DNA der VH- und/oder VL-Regionen durch RT-PCR, wobei
i. die DNA der VH- und/oder VL-Regionen aus einer Zelle erhalten wurde, die aus einer einzelnen Vertiefung isoliert wurde, die in Schritt d) des Verfahrens nach einem der Ansprüche 1 bis 12 identifiziert wurde, oder
ii. die DNA der VH-Region aus einer Zelle erhalten wurde, die aus einer einzelnen Vertiefung isoliert wurde, die in Schritt d) des Verfahrens nach einem der Ansprüche 2 bis 12 identifiziert wurde, und die DNA der VL-Region aus einer Zelle isoliert wurde, die aus einer anderen Vertiefung isoliert wurde, die auf dem gleichen Array in Schritt d) des Verfahrens nach einem der Ansprüche 2 bis 12 identifiziert wurde.

14. Verfahren zum Erhalten einer Zelle, die einen antigenspezifischen monoklonalen Antikörper produziert, umfassend die folgenden Schritte:
a) Isolieren von mRNA aus einer Zelle, die in den identifizierten Vertiefungen von Schritt d) des Verfahrens der Ansprüche 1 bis 12 zugegen ist, oder aus einer Zellkultur, die aus einer solchen Zelle erhalten wurde;
b) Durchführen einer reversen Transkription der mRNA und Amplifizieren der entsprechenden cDNA durch RT-PCR;
c) Klonieren der DNA-Sequenzen, die den VH- und VL-Regionen entsprechen, in einen geeigneten Expressionsvektor.

15. Verfahren zur Herstellung eines monoklonalen Antikörpers, umfassend die folgenden Schritte:
a) Durchführen des Verfahrens nach einem der Ansprüche 1 bis 12 zum Identifizieren einer Zelle, die einen Antikörper gegen ein Zielantigen sezerniert;
b) Isolieren der VH- und VL-Gene, die den Antikörper codieren, aus der Zelle von Schritt a) oder ihrer Zellkultur und Klonieren dieser Gene; und
c) Exprimieren der Gene von Schritt b) in einer geeigneten Zelle zum Erhalt des monoklonalen Antikörpers.

## Revendications

1. Procédé d'identification, dans une population de cellules comprenant des cellules sécrétrices d'anticorps, d'une cellule sécrétant un anticorps contre un ou plusieurs antigène(s) cible(s), ledit procédé comprenant les étapes de :
a) stimulation et amplification *in vitro* des cellules de ladite population et échantillonnage de cellules afin d'obtenir de multiples groupes de cellules qui contiennent des clones cellulaires expansés ;
b) criblage des surnageants desdits groupes de cellules pour identifier et sélectionner les groupes de cellules contenant des cellules sécrétant des anticorps se liant au(x)dit(s) antigène(s) cible(s) et/ou présentant une activité biologique ;
c) dépôt des cellules desdits groupes de cellules identifiés dans un réseau comprenant des puits multiples dans des conditions telles qu'au plus trois cellules soient présentes dans chaque puits dudit réseau ;
d) culture des cellules dans les puits dudit réseau et criblage de celles-ci pour identifier les puits contenant une cellule sécrétant un anticorps contre le(s)dit(s) antigène(s) cible(s).

2. Procédé selon la revendication 1, dans lequel, dans l'étape c), toutes les cellules qui sont présentes dans un réseau donné proviennent du même groupe de cellules.

3. Procédé selon l'une quelconque des revendications 1 ou 2, dans lequel ladite population de cellules a été infectée par un virus lymphotrope avant ou pendant l'étape a).

4. Procédé selon l'une quelconque des revendications 1 à 3, dans lequel, après l'échantillonnage de cellules, lesdits groupes de cellules sont cultivés dans un milieu de culture dans des conditions dans lesquelles les cellules sécrètent des anticorps dans ledit milieu de culture.

5. Procédé selon l'une quelconque des revendications 1 à 4, dans lequel, dans l'étape b), les surnageants de groupes de cellules sont sélectionnés pour la présence d'un anticorps se liant auxdits un ou plusieurs antigènes cibles et pour la présentation d'une activité biologique.

6. Procédé selon l'une quelconque des revendications 1 à 4, dans lequel, dans l'étape b), les surnageants de groupe de cellules sont sélectionnés pour la présence d'un anticorps ayant un profil de liaison d'intérêt en conduisant des essais de liaison multiples.

7. Procédé selon l'une quelconque des revendications 1 à 6, dans lequel la taille et la forme des puits dudit réseau dans l'étape c) permettent l'entrée d'une seule cellule par puits.

8. Procédé selon l'une quelconque des revendications 1 à 7, dans lequel ledit réseau dans l'étape c) comprend une couche de revêtement d'une substance de liaison sur au moins la surface autour des puits, dans lequel ladite substance de liaison a la capacité à se lier à au moins une partie d'un anticorps sécrété et dans lequel les anticorps sécrétés peuvent diffuser et se lier à ladite substance de liaison.

9. Procédé selon l'une quelconque des revendications 1 à 8, dans lequel l'étape d) comprend l'étape de production d'une empreinte du réseau sur un support solide et de détection de la présence de l'anticorps se liant à l'antigène sur ledit support solide.

10. Procédé selon l'une quelconque des revendications 1 à 9, dans lequel ladite population de cellules comprenant des cellules sécrétrices d'anticorps provient d'un être humain.

11. Procédé selon la revendication 8, dans lequel ladite substance de liaison est un ou plusieurs dudit/desdits antigène(s) cible(s) ou un anticorps anti-immunoglobuline.

12. Procédé selon l'une quelconque des revendications 1 à 11, comprenant en outre l'étape indépendante d'extraction de(s) cellule(s) unique(s) à partir des puits identifiés dans l'étape d).

13. Procédé de récupération de l'ADN des régions VH et/ou VL d'un anticorps monoclonal spécifique pour un antigène comprenant les étapes de :
a) mise en oeuvre du procédé selon l'une quelconque des revendications 1 à 12 afin d'identifier une cellule sécrétant un anticorps contre un antigène cible ; et
b) obtention par RT-PCR de l'ADN des régions VH et/ou VL dans lequel
i. l'ADN des régions VH et VL a été obtenu à partir d'une cellule isolée à partir d'un puits unique identifié dans l'étape d) du procédé selon l'une quelconque des revendications 1 à 12, ou
ii. l'ADN de la région VH a été obtenu à partir d'une cellule isolée à partir d'un puits identifié dans l'étape d) du procédé de l'une quelconque des revendications 2 à 12 et l'ADN de la région VL a été isolé à partir d'une cellule isolée à partir d'un autre puits identifié sur le même réseau, dans l'étape d) du procédé selon l'une quelconque des revendications 2 à 12.

14. Procédé d'obtention d'une cellule produisant un anticorps monoclonal spécifique pour un antigène comprenant les étapes de :
a) isolement d'ARNm à partir d'une cellule qui est présente dans les puits identifiés de l'étape d) du procédé des revendications 1 à 12, ou à partir d'une culture de cellules obtenue à partir d'une telle cellule ;
b) mise en oeuvre d'une transcription inverse dudit ARNm et amplification de l'ADNc correspondant par RT-PCR ;
c) clonage desdites séquences d'ADN correspondant aux régions VH et VL dans un vecteur d'expression adapté.

15. Procédé de production d'un anticorps monoclonal comprenant les étapes de :
a) mise en oeuvre du procédé selon l'une quelconque des revendications 1 à 12 pour identifier une cellule sécrétant un anticorps contre un antigène cible ;
b) isolement des gènes VH et VL codant pour l'anticorps à partir de la cellule de l'étape a) ou sa culture de cellules et clonage de ces gènes ; et
c) expression des gènes de l'étape b) dans une cellule adaptée pour obtenir l'anticorps monoclonal.
